(19)

**Europäisches Patentamt**
**European Patent Office**
**Office européen des brevets**

(11) **EP 3 989 878 B1**

(12) **EUROPEAN PATENT SPECIFICATION**

(45) Date of publication and mention
of the grant of the patent:
**28.01.2026 Bulletin 2026/05**

(21) Application number: **20743078.6**

(22) Date of filing: **29.06.2020**

(51) International Patent Classification (IPC):
***A61F 2/12*** *(2006.01)*    ***A61M 5/142*** *(2006.01)*

(52) Cooperative Patent Classification (CPC):
**A61F 2/12; A61M 5/14276; A61N 1/05;**
**A61N 1/375;** A61F 2210/0057; A61F 2250/0003;
A61K 9/0009; A61K 9/0024; A61M 5/14593

(86) International application number:
**PCT/EP2020/068316**

(87) International publication number:
**WO 2020/260724 (30.12.2020 Gazette 2020/53)**

(54) **AN IMPLANTABLE MEDICAL DEVICE**

IMPLANTIERBARE MEDIZINISCHE VORRICHTUNG

DISPOSITIF MÉDICAL IMPLANTABLE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.06.2019 US 201962867927 P**
**23.07.2019 EP 19187842**

(43) Date of publication of application:
**04.05.2022 Bulletin 2022/18**

(73) Proprietors:
• **University of Galway**
**Galway (IE)**
• **Massachusetts Institute of Technology**
**Cambridge, MA 02139 (US)**

(72) Inventors:
• **ROCHE, Ellen**
**Cambridge, MA 02141 (US)**
• **DOLAN, Eimear**
**Pollbawn Hollymount, Co. Mayo (IE)**

• **DUFFY, Garry**
**Galway H91 DVF7 (IE)**
• **ROTHENBUCHER, Sandra**
**80804 Munich (DE)**
• **WHYTE, William**
**Longford Aherlow, Co. Tipperary E34 H248 (IE)**
• **VARELA, Claudia**
**Mission, TX 78574 (US)**
• **ROBINSON, Scott**
**Galway, H91 V32F (IE)**
• **MENDEZ, Keegan**
**Cambridge, MA 02139 (US)**

(74) Representative: **Purdylucey Intellectual Property**
**23 Ely Place**
**Dublin 2 D02 N285 (IE)**

(56) References cited:
EP-A1- 2 900 212          US-A- 4 718 893
US-A- 5 707 361           US-A1- 2006 069 403
US-A1- 2009 177 157       US-A1- 2014 236 210
US-A1- 2014 371 674       US-A1- 2015 126 968
US-A1- 2016 331 956       US-B2- 9 724 189

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the Invention

The present invention relates to an implantable medical device, in particular an implantable medical device modified to reduce fibrotic encapsulation. Also contemplated but not forming part of the invention are methods of using the device, including methods of reducing fibrotic encapsulation of implantable medical devices

Background to the Invention

The long-term performance of implantable medical devices is drastically limited by complex and unpredictable foreign body responses (FBR). Medical devices that depend on an interface with native tissue are particularly vulnerable, such as neural probes [1, 2], indwelling catheters [3], mammary implants [4], pacemakers [5], glucose biosensors [6-8] and drug and cell delivery devices [6, 9-12]. Such devices are often subject to a collection of biological host responses such as fibrosis, bacterial biofilm formation and inflammation, which can impair functionality. At present, device failure is expected and inevitable, and the costs, inconvenience and morbidity that it can impose on patients are largely accepted.

The implantable medical devices market is mature and growing (compound annual growth rate of 8%), with an estimated worth of approximately 100 billion US dollars in 2019. Implantable medical devices have various failure rates that can be attributed to fibrosis, and can be as high as 30-50% for implantable pacemakers and 30% for mammoplasty prosthetics.

Once a device is implanted a complex series of events is initiated to protect the host from the 'foreign body'. Shortly after implantation, fibrinogen and other proteins bind to the surface of the device/foreign body, macrophages then bind and, over time, become multinucleated giant cells releasing inflammatory cytokines. In response to these signals, quiescent fibroblasts are transformed into myofibroblasts, which synthesize procollagen. The procollagen becomes crosslinked and this mature crosslinked collagen and other extracellular matrix proteins contribute to the formation of a dense fibrous capsule that becomes impermeable or hypo-permeable to many compounds. Various strategies have been investigated to mitigate the FBR, such as generating scaffolds that release small molecules, surface chemistry modifications, and modifying implant size and geometry. However, these treatments have met with limited success because of difficulty in incorporating these modifications in a range of implantable devices.

US2014236210 describes a device for tissue expansion with an implantable portion which comprises a gas source in communication with an expandable chamber. The gas source will release gas for a gradual inflation of the expandable chamber. The tissue expander can be used for creating a pocket within breast tissue.

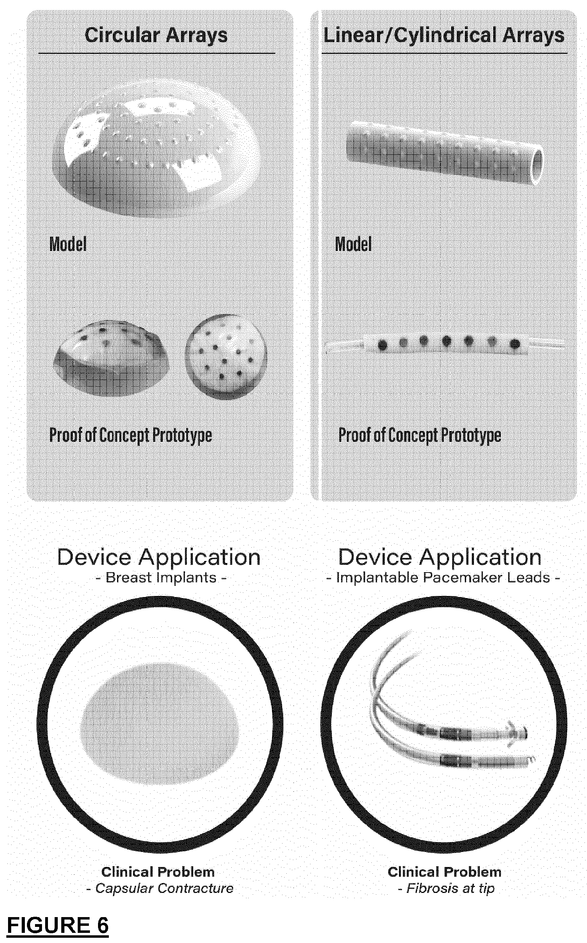

US4718893 and US5707361 describe implantable drug infusion devices. The device of US4718893 has a chamber for drug and a separate pressure-balancing chamber. When the device is filled with drug, the rigid base (26) moves outwardly and when drug is slowly infused the base moves gradually inwardly. The walls of the device are rigid but resiliently flexible to provide a spring effect, and are configured for slow gradual movement. The device of US5707361 (Fig. 6) has an annular expansion chamber (5) with a flexible polymer wall (40). The purpose of this chamber is to allow the drug chamber (6) expand when it is filled with drug. The wall (40) moves outwardly when the drug chamber is filled and move gradually inwardly as drug is infused. Both devices are prone to fibrotic encapsulation while implanted in the body.

It is an object of the invention to overcome at least one of the above-referenced problems.

Summary of the Invention

Throughout the disclosure, when the unit PSI is used, one PSI is approximately 6,895 Pascal (N/m2).

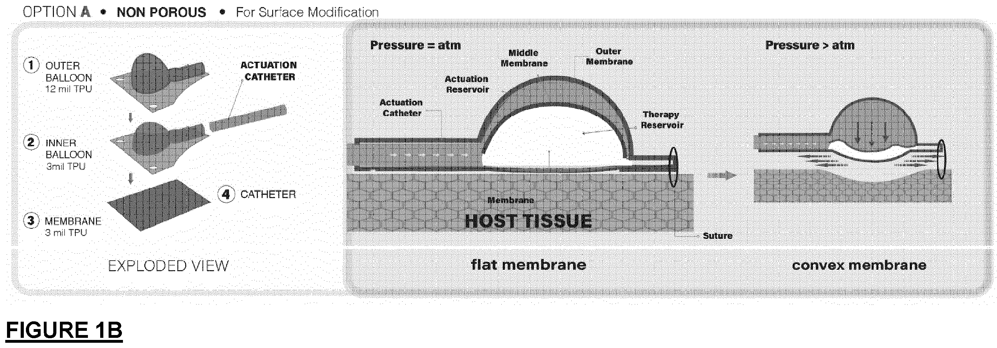

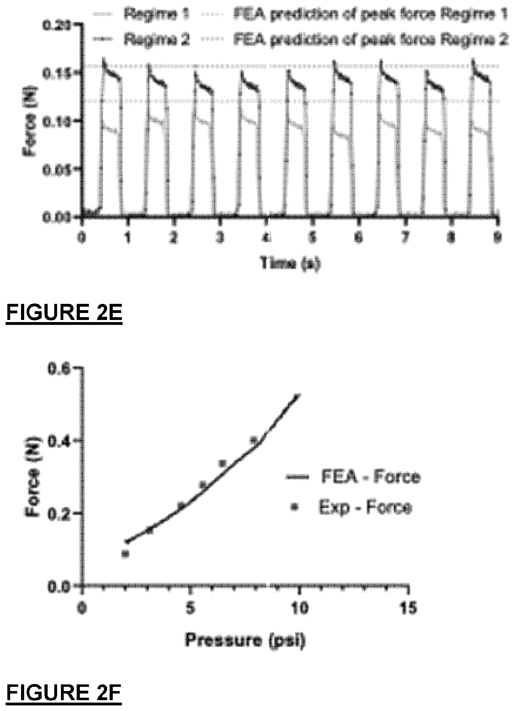

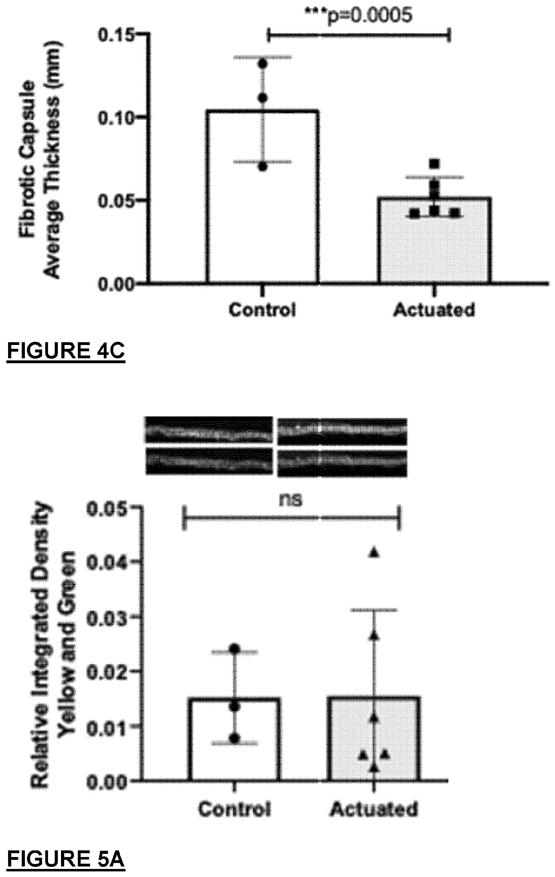

The Applicant has addressed the problem of fibrotic encapsulation of implanted medical devices by providing a soft tissue interfacing surface of the medical device with one or more and preferably an array of soft robotic capsules (Fig. 1B) that can be actuated to cause cyclical deflection of a soft tissue interfacing membrane (Fig. 2E) of the or each capsule that actively modulates the biomechanics of the biotic-abiotic interface by altering strain, fluid flow and cellular activity in the peri-implant tissue. The Applicant has shown that cyclical actuation of soft robotic capsules on a medical device implanted in a preclinical rodent model resulted in a significant reduction in fibrous capsule thickness (Fig. 4C) p=0.0005) compared to non-actuated controls, while the collagen density and orientation were not changed (p>0.05). A significant reduction in myofibroblasts (p=0.0036) in the actuated group was also demonstrated. Computational models quantified the effect of actuation on the reservoir and surrounding fluid. In a further aspect, adding a porous membrane (or a membrane that becomes transiently porous only upon actuation/deflection, or renders the native foreign body response porous) and a therapy reservoir to device of the invention, resulted in increased transport of a drug analog and enhanced pharmacokinetics and time to functional effect of an inotropic agent. The Applicant also shows that the capsule of the invention can push drug through a fibrotic capsule into surrounding tissue (Fig. 12).

In a first aspect, the invention provides an implantable medical device having a soft tissue interfacing surface comprising at least one, and preferably a plurality of, actuatable capsule(s). The or each capsule comprises a soft tissue interfacing deflectable membrane configured for cyclical deflection when the capsule is actuated to modulate the biomechanics of the soft tissue interface, typically by altering one or more of displacement, force, strain, fluid flow and cellular activity in peri-implant tissue at the soft tissue interface.

The actuatable capsule may be configured for mechanical or electrical actuation, for example by inflation (i.e. cyclical inflation) to cause deflection of the soft tissue interfacing deflectable membrane. Alternative methods of mechanical actuation of the capsule are envisaged. For example, ultrasound may be employed to cause deflection of the soft tissue interfacing deflectable membrane. The capsule may include an ultrasound emitter configured to generate a shock wave in the fluid in the capsule by cavitation, leading to deflection of the deflectable membrane. Alternatively, an external ultrasound emitter may be employed to actuate the capsule. In another embodiment, part of the capsule (for example the soft tissue interfacing deflectable membrane) may comprise an actuating material (for example a charged material) that is configured to actuate the capsule when electrically stimulated. The actuating material may be a piezoelectric material. The deflectable membrane may comprise actuating material.

The actuatable capsule typically comprises:

an actuation chamber containing a first fluid;
a therapeutic chamber containing a second fluid that optionally may be replenishable from an external reservoir or transcutaneously;
a deflectable membrane separating the actuation chamber and therapeutic chamber; and
actuation means in fluidic communication with the actuation chamber for actuation of the actuation chamber,

wherein the therapeutic chamber comprises the soft tissue interfacing deflectable membrane.

The use of an actuation chamber and a separate therapeutic chamber has been found to allow better control of the defection of the soft tissue interfacing deflectable membrane, as the actuation chamber is generally not in contact with surrounding tissue. In addition, the Applicant has shown that in this embodiment the deflection of the membrane between the therapeutic and actuation chamber can push drug through the porous deflectable membrane and a fibrotic capsule, and into surrounding tissue (Fig. 12).

In one embodiment, the actuation means comprises an actuation conduit in fluidic communication with the actuation chamber for fluidic actuation of the actuation chamber,

In one embodiment, the actuation chamber is defined by a non-deflectable membrane and the deflectable separating membrane.

In one embodiment, the therapeutic chamber is defined by the deflectable separating membrane and the soft tissue interfacing deflectable membrane.

In one embodiment, the capsule is a millisized capsule. However, it will be appreciated that larger or small capsules may be employed, for example micron-sized capsules or centimetre-sized capsules.

In one embodiment, the soft tissue interfacing surface comprises a plurality of capsules, for example at least 5, 10, 15, 20, 25, 30, or 40. Preferably, the plurality of capsules is arranged in an array.

In one embodiment, the soft tissue interfacing surface of the medical device comprises (e.g. is provided by) a skin that can optionally be retro-fitted to the implantable medical device. The skin can cover all or part of a tissue interfacing surface of the implantable medical device. The skin can be pre-shaped to conform closely to the soft tissue interface. For example, the skin can be shaped to conform to a surface of a tissue implant, for example a mammary implant. The skin can also be provided in the form of a sheath, i.e. configured to conform to a conduit implant (for example an indwelling catheter, pacemaker leads, or a neural implant). It will be appreciated that the skin can be configured to cover all or only a part of the medical device, and can be attached to the medical device during manufacture or retro-fitted to the implant prior to use.

In one embodiment, the or each capsule is integrally formed on a surface of the medical device.

In one embodiment, the soft tissue interfacing deflectable membrane is disposed on the soft tissue interfacing surface with the soft tissue interfacing deflectable membrane substantially flush with the soft tissue interfacing surface.

In one embodiment, the or each capsule is dome shaped having a substantially flat base provided by the soft tissue interfacing deflectable membrane. It will be appreciated that the capsule may have other shapes, for example cylindrical or rectangular or any other shape.

In one embodiment, the or each capsule comprises a dome shaped actuation chamber and a smaller dome shaped therapeutic chamber disposed within the actuation chamber.

In one embodiment, the deflectable membrane is non-porous. However, in some embodiments, it may be porous (e.g. porous to active agent contained within the second fluid), or may be actuated to become porous (for example stretched or deflected). This embodiment allows the capsule perform two functions, inhibition of fibrotic capsule formation around the medical device, and drug delivery during actuation in which the cyclical deflection of the soft-tissue interfacing membrane can push drug into surrounding tissue, including pushing drug through any fibrotic capsule material. In one embodiment, the deflectable membrane is configured to be non-porous in a resting stage, and become porous when deflected. Thus, in one embodiment, the or each capsule comprises a therapeutic conduit in fluidic communication with the capsule (in one embodiment in fluidic communication with the therapeutic chamber) for delivery of an active agent (the active agent may be therapeutic or diagnostic), and in which the soft tissue interfacing deflectable membrane is typically porous or configured to become porous when it is actuated/deflected. This allows drug in the capsule/therapeutic chamber to be delivered by diffusion or by pumping. The use of a deflectable membrane that becomes porous only during actuation/deflection, allows for intermittent drug release.

In one embodiment, the or each capsule comprises a second actuation conduit in fluidic communication with the actuation chamber. This allows the capsule to be connected in series to a pump, with fluid being pumped into the actuation chamber through one conduit and exiting the chamber through the second conduit.

In one embodiment, the device comprises an implantable pump operatively connected to the first and/or second actuation conduit. While the use of an implantable pump is preferred, it will nonetheless be appreciated that a remote, non-implantable pump may be employed to fluidically actuate the actuation chamber and cause cyclical deflection of the soft tissue interfacing membrane. In one embodiment, the pump is a piezoelectric pump. The pump may be configured to provide pulsative flow in the actuation conduit.

When a plurality of capsules is employed, the pump may be operatively connected to a plurality of the capsules. The pump may be connected to the plurality capsules in series or in parallel.

In one embodiment, the pump is operatively connected to the first and second conduits in series and is configured to pump fluid to the first conduit and receive fluid from the other conduit.

In one embodiment, the second conduit comprises a fluidic resistor configured to resist fluid flow. This helps with actuation of the actuation chamber by helping maintain pressure in the actuation chamber when the pump is actuated.

In one embodiment, the second conduit comprises a fluidic capacitor typically disposed distally of the fluidic resistor (i.e an expandable chamber distal of the fluidic resistor that helps release pressure in the first fluid and allow deflation of the actuation chamber).

In one embodiment, the implantable medical device is selected from a tissue implant, an indwelling catheter, a pacemaker, a biosensor, a drug or cell delivery device, and a neural probe. The or each capsule may be integrally formed as part of a soft tissue interfacing surface of the implant, or may be provided as a "skin" that can be attached to all or part of the soft tissue interfacing surface of the medical device during manufacture, or retro-fitted after manufacture and prior to implantation.

In any embodiment, the device of the invention includes a passive drug infusion capsule. In one embodiment, the passive drug infusion capsule may be disposed adjacent to an actuatable capsule.

In another aspect, the invention provides a retro-fit skin for use with an implantable medical device to form an implantable medical device of the invention. The retro-fit skin is configured to adhere to and cover at least part of a surface of the implantable medical device and form a soft tissue interfacing surface on the medical device, and comprises at least one inflatable capsule. The capsule typically comprises:

> an actuation chamber containing a first fluid;
> a therapeutic chamber containing a second fluid that can optionally be replenished from an internal reservoir or transcutaneously;
> a deflectable membrane separating the actuation chamber and therapeutic chamber; and
> actuation means in fluidic communication with the actuation chamber for actuation of the actuation chamber,
> wherein the therapeutic chamber comprises a soft tissue interfacing deflectable membrane.

In one embodiment, the actuation means comprises an actuation conduit in fluidic communication with the actuation chamber for fluidic actuation of the actuation chamber,

In one embodiment, the skin is formed from a soft polymeric film, for example a silicone skin.

In one embodiment, the actuation chamber is defined by a non-deflectable membrane and the deflectable separating membrane.

In one embodiment, the therapeutic chamber is defined by the deflectable separating membrane and the soft tissue interfacing deflectable membrane.

In one embodiment, the capsule is a millisized capsule.

In one embodiment, the skin comprises an array of capsules.

The skin can cover all or part of a tissue interfacing surface of the implantable medical device. The skin can be pre-shaped to conform closely to the soft tissue interface. For example, the skin can be shaped to conform to a surface of a tissue implant, for example a mammary implant. The skin can be provided in the form of a sheath, i.e. configured to conform to a conduit implant (for example an indwelling catheter, pacemaker leads, or a neural implant). It will be appreciated that the skin can be configured to cover all or only a part of the medical device, and can be attached to the medical device during manufacture or retro-fitted to the implant prior to use.

In one embodiment, the or each capsule is integrally formed on a surface of the medical device.

In one embodiment, the soft tissue interfacing deflectable membrane is disposed on the skin with the soft tissue interfacing deflectable membrane substantially flush with the soft tissue interfacing surface of the skin.

In one embodiment, the or each capsule is dome shaped having a flat base provided by the soft tissue interfacing deflectable membrane. It will be appreciated that the capsule may have other shapes, for example cylindrical or rectangular or any other shape.

In one embodiment, the or each capsule comprises a dome shaped actuation chamber and a smaller dome shaped therapeutic chamber disposed within the actuation chamber.

In one embodiment, the or each capsule comprises a therapeutic conduit in fluidic communication with the therapeutic chamber for delivery of an active agent (the active agent may be therapeutic or diagnostic), and in which the soft tissue interfacing deflectable membrane is porous (or actuatable to become porous). In this manner, deflection of the porous membrane causes an increase in transport of the active agent into the surrounding tissue.

In one embodiment, the or each capsule comprises a second actuation conduit in fluidic communication with the actuation chamber. This allows the capsule to be connected in series to a pump, with fluid being pumped into the actuation chamber through one conduit and exiting the chamber through the second conduit.

In one embodiment, the device comprises an implantable pump operatively connected to the first and/or second actuation conduit. While the use of an implantable pump is preferred, it will nonetheless be appreciated that a remote, non-implantable pump may be employed to pneumatically actuate the actuation chamber and cause cyclical deflection of the soft tissue interfacing membrane.

In one embodiment, the pump is operatively connected to the first and second conduits in series and is configured to pump fluid to the first conduit and receive fluid from the other conduit.

In one embodiment, the second conduit comprises a fluidic resistor configured to resist fluid flow. This helps with actuation of the actuation chamber by helping maintain pressure in the actuation chamber when the pump is actuated.

In one embodiment, the second conduit comprises a fluidic capacitor (i.e an expandable chamber distal of the fluidic resistor that helps release pressure in the first fluid and allow deflation of the actuation chamber).

The invention also provides an implantable medical device comprising a retro-fit skin of the invention adhered to at least a part of the soft tissue facing surface of an implantable medical device.

Also described is a method (typically a method of reducing fibrotic encapsulation of an implantable medical device), comprising the steps of:

implanting an implantable medical device according to the invention into a subject with the soft tissue interfacing surface of the device in contact with or adjacent to soft tissue of the subject; and
cyclically actuating the or each actuatable capsule disposed on the soft tissue interfacing surface to effect cyclical deflection of the soft tissue interfacing membrane,
whereby the cyclical deflection of the soft tissue interfacing membrane modulates the biomechanics of the soft tissue interface by altering one or more of displacement, force, strain, fluid flow and cellular activity in peri-implant tissue at the soft tissue interface.

In one embodiment, the method comprises cyclically actuating the actuation chamber to effect cyclical deflection of the soft tissue interfacing membrane.

In one embodiment, the device or actuation chamber is actuated (or configured for actuation) at an actuation pressure of 0.5 to 20.0 PSI, 0.5 to 10.0 PSI, 0.5 to 5.0 PSI, and preferably 0.5 to 3.0 PSI

In one embodiment, the device or actuation chamber is actuated (or configured for actuation) at a frequency of 0.1 to 10.0 Hz, preferably 0.5 to 5 Hz, and more preferably 0.5 to 1.5 Hz.

In one embodiment, the cyclical actuation of the actuation chamber is performed continuously or periodically, for example once every hour, day, week or month. For example, the capsule may be actuated for five minutes every five hours, and the 5 minute actuation period may comprise cyclical deflection of the soft-tissue interfacing membrane at a frequency of 0.1 to 10 Hz.

In one embodiment, the or each capsule comprises a therapeutic conduit in fluidic communication with the therapeutic chamber for delivery of an active agent (the active agent may be therapeutic or diagnostic), and in which the soft tissue interfacing deflectable membrane is typically porous or configured to be transiently porous during actuation/-deflection, wherein the method includes a step of delivering or pumping active agent into the therapeutic chamber to refill or replenish the chamber.

In one embodiment, the implantable medical device is a cell encapsulation device.

The invention also provides a method of making a device of the invention. Device manufacture procedure typically comprises two steps - balloon thermoforming and heat sealing. A large balloon and smaller balloon are generally formed, typically by thermoforming. Balloon Thermoforming typically involves the steps of: 1) A substrate (i.e. Thermoplastic urethane (TPU) sheets) (for example one for inner (0.07 mm balloon) and one for outer (0.3 mm) balloon)) were mounted separately in a thermal former with a vacuum platform and a positive 3D printed mould was placed on the platform, 2) The substrate sheet was secured in place, 3) The substrate was brought close to the heating element, 4) the heat was turned on, 5) the substrate was heated until it sags in the centre, 6) the platform was lowered over the positive mould, 6) the vacuum was applied to form the sheet over the positive mould, 8) the formed substrate was removed from the positive mould. Heat sealing generally involves: 1) The formed substrate was cut out, 2) the larger formed substrate (outer balloon) was placed in a 3D printed negative mould, 3) the smaller formed substrate (inner balloon) was placed in a 3D printed negative mould on top of outer balloon, 4) A non-stick material (i.e. a teflon strip) was inserted into the groove of the negative mould between relevant layers of substrate to keep the catheter channels open during the following heat-sealing 5) a membrane (porous or non-porous) was placed on top and a layer of non-stick material was played on top to protect the assembly, 5) the arm of the heat press was brought down on top of the mould, 6) the assembly was heat sealed with a heat transfer machine, 7) the heat arm and non-stick material was removed, 8) device reservoir was complete. Catheter tubing was inserted into relevant channels and heat shrink tubing was used to seal the devices.

Other aspects and preferred embodiments of the invention are defined and described in the other claims set out below.

Brief Description of the Figures

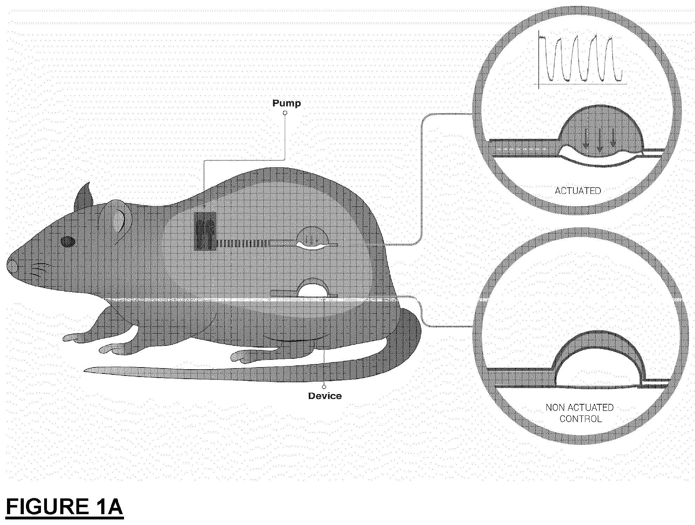

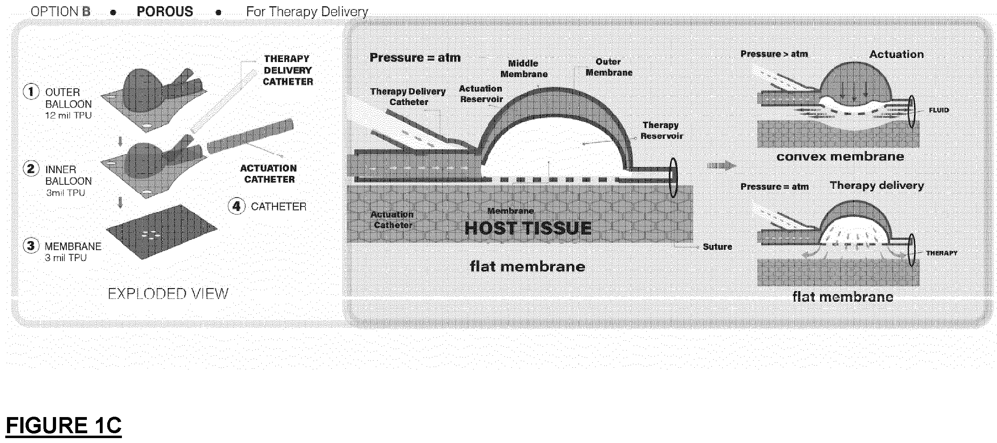

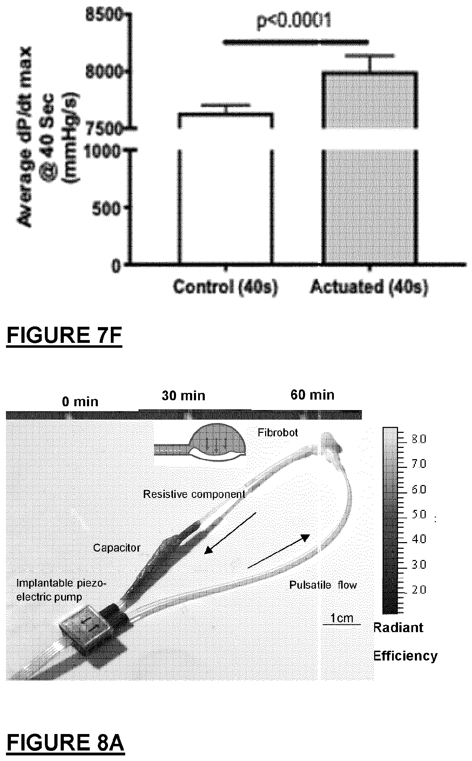

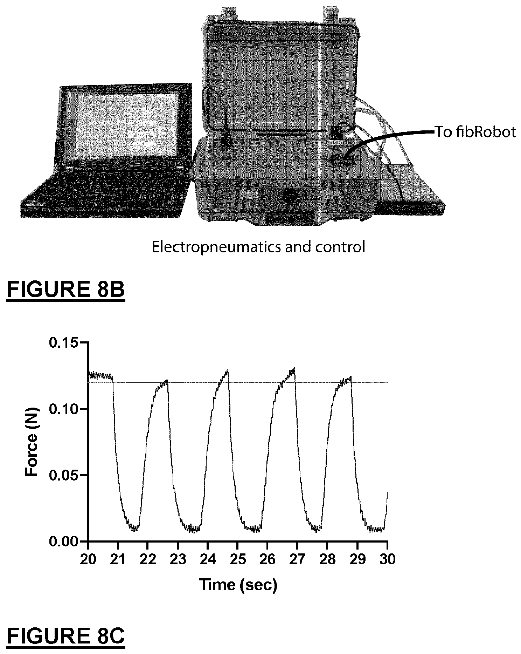

**Figure** 1: An overview of the device and the proposed mechanism of action. A) Implantable system showing a side-by-side implantation of a control and actuation group. B) Non-porous configuration for foreign body modulation for implantable devices. C) Porous configuration for therapy delivery. The device can be cyclically actuated by an implantable piezoelectric pump, and we demonstrate this in a rat model (Fig. 1A, Fig. 8). Cyclical pressurization results in a predictable, regime-specific deflection and strain of the lower functional membrane, see Fig. 1B and C. Multiple such reservoirs can be incorporated into a thin, conformable matrix that can be designed to be part of, or surround, implantable devices to reduce the host FBR. The proposed mechanism of action of the individual reservoirs is shown in Fig. 1B and C. When the actuation reservoir is pressurized through the actuation line, the volume, and then pressure of the chamber increases, causing a downward deflection of the middle, and subsequently the lower membrane. Actuation causes deflection and strain of the membrane, and results in fluid flow at the tissue interface (blue arrows Fib. 1B and C) which is proposed to interfere with cell activity and initiation of the fibrotic response. Two configurations of the device are described - one version with an impermeable, non-porous lower membrane (Fig. 1B), which would be incorporated in a sheath as a surface modification (suited toward implants with a structural role), and a second version with a permeable, porous membrane (Fig.1C) that incorporates an additional therapy delivery line to the lower reservoir to allow transport of therapy into the tissue (suited for drug delivery or biosensing applications).

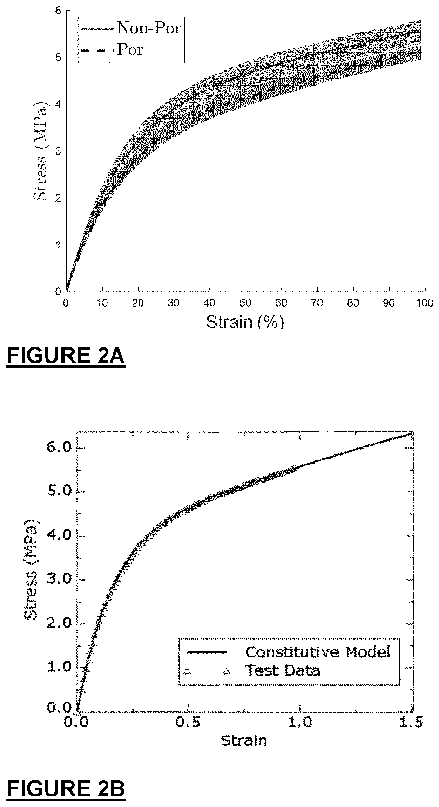

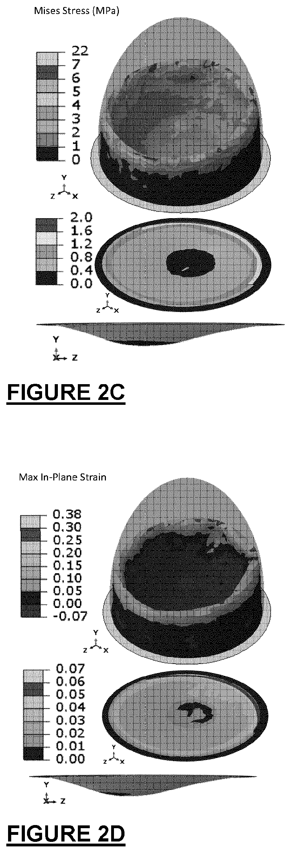

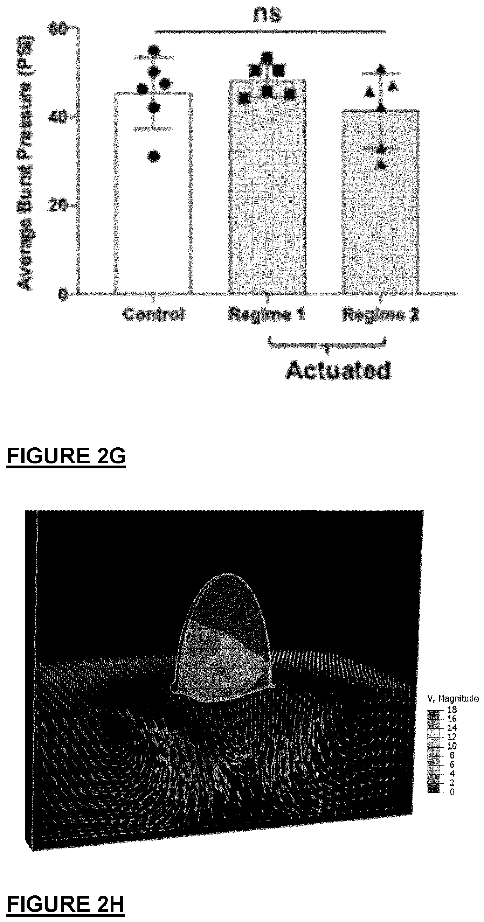

**Figure 2:** Computational and experimental characterization: A) Stress/strain plots for porous and non-porous thermoplastic urethane specimens. B) Fitting of the porous data to the Ogden hyperelastic model. C) Mises stress contour plots for the overall device, and the lower membrane (top-down and side view). D) Maximum in-plane strain for the overall device, and the lower membrane (top-down and side view). E) Experimental cyclical force measurements two actuation regimes (regime 1 = 1 PSI at 1 Hz, regime 2 = 2 PSI at 1 Hz), and finite element predicted force for each. F) Relationship between input pressure and actuation force as measured experimentally, and predicted with finite element analysis (FEA). G) Burst failure of device of the invention reservoir after actuating at regime 1 or regime 2 for 100,000 cycles. n=6/group, data are mean $\pm$ standard deviation p>0.05. H) Smooth particle hydrodynamic model showing the direction and magnitude of fluid flow surrounding device of the invention during actuation.

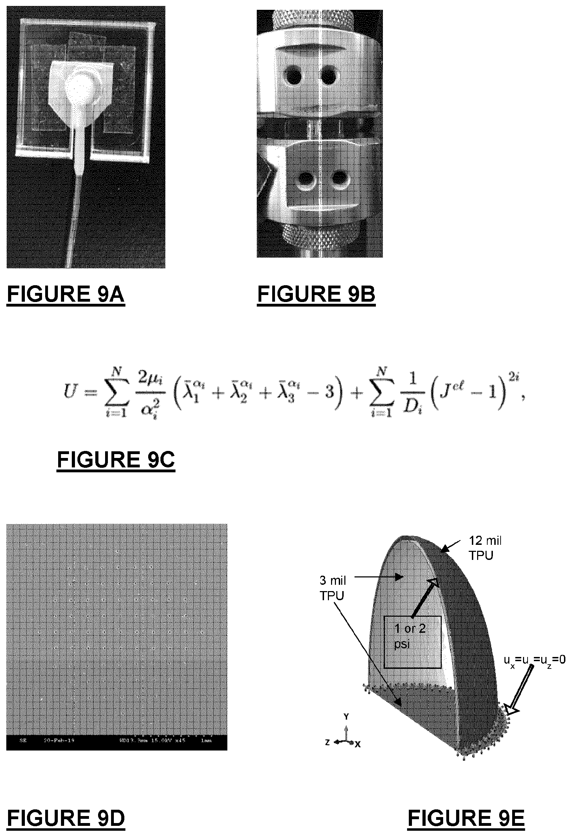

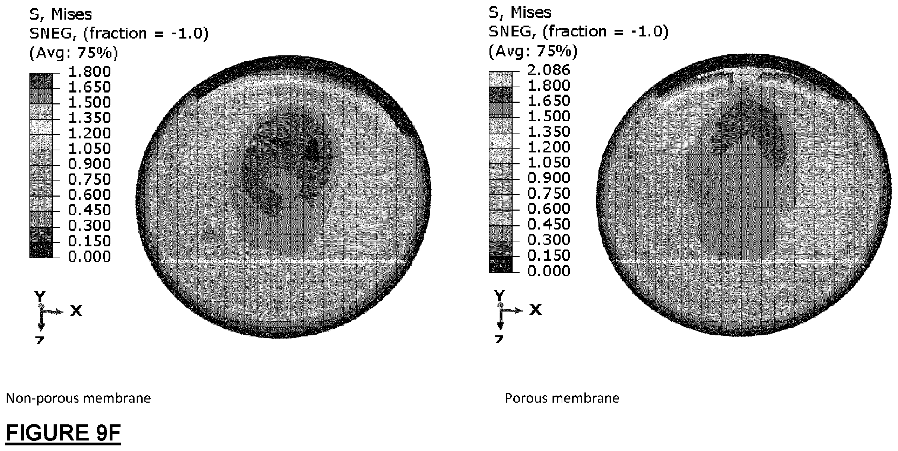

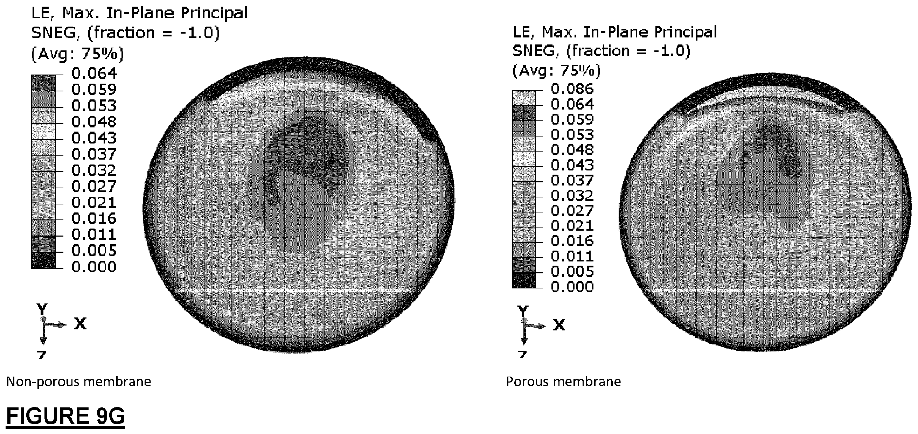

The thermoplastic urethane used for the membrane (porous and non-porous) was tensile tested according to ASTMD638. The resulting stress vs strain plots are shown in Fig. 2A. The data was fitted to a hyperelastic material model with the Ogden model being selected as the best fit (Fig. 2B, Fig. 9). With this material model as an input, a finite element analysis model was created to demonstrate the overall strain (Fig. 2C), and resulting stress (Fig.2D) in the inner and outer balloons and membrane. This was repeated for the non-porous and porous membranes with maximum in-plane strain of 6.4% and 8.6% respectively (Fig. 9). To validate the computational models, we measured the force that the membrane applies to interfacing tissue, using a custom acrylic holder (Fig. 9) to consistently support the device in a materials tester, with the functional membrane in contact with the upper load cell. The cyclical forces for two regimes (regime 1 = 1 PSI at 1 Hz, regime 2 = 2 PSI at 1 Hz) were recorded, and peak forces were compared to each other, and their corresponding finite element models, showing an excellent match between measured and predicted values (Fig. 2E). To further characterize the possible range of input pressures, higher pressures and corresponding forces were also modelled computationally and experimentally (Fig. 2F).

A one-way analysis of variance (ANOVA) was performed with post hoc Tukey's test to compare the burst pressure of the devices after they were subjected to 100,000 cycles at pressures of regime 1 or regime 2. There was no statistical difference between regime 1 (P = 0.7685) or regime 2 (P = 0.6251) compared with the non-actuated control (Fig. 2G) indicating that the applied actuation regimes fall within the fatigue limit of the devices. Finally, we used a smooth particle hydrodynamics model to quantify the fluid perturbations from device actuations during device actuation in fluid (Fig. 2H).

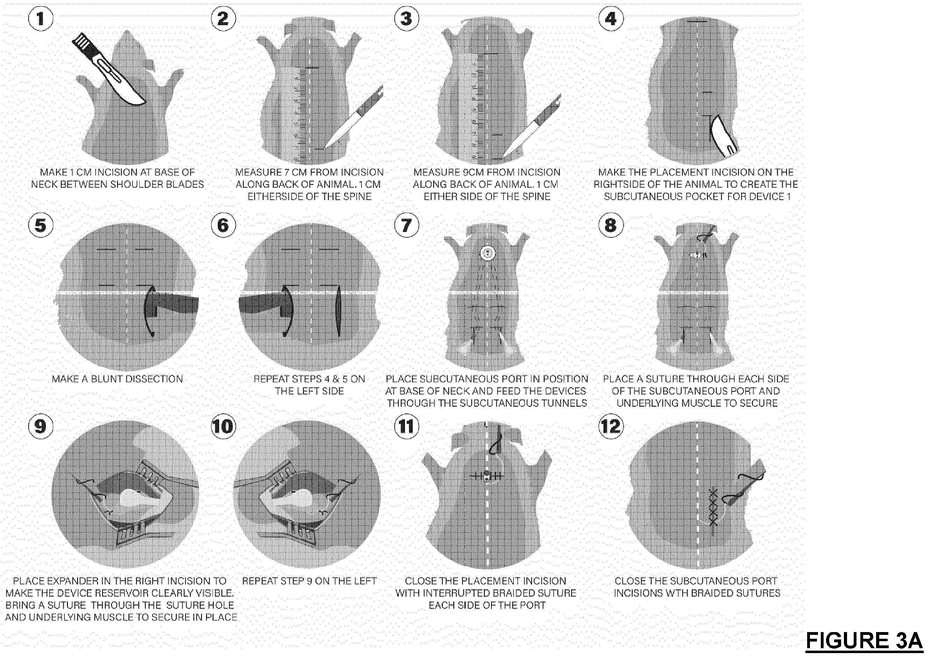

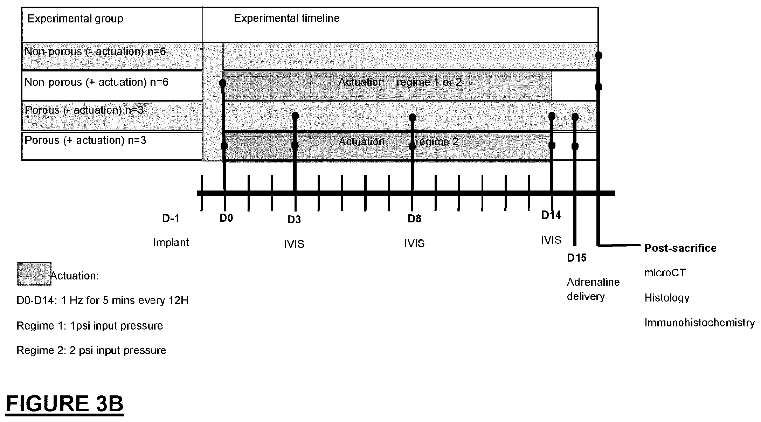

**Figure** 3: Pre-clinical implementation of device of the invention. A) Device implantation procedure. Each animal has two implanted reservoirs subjected to no actuation, regime 1 or regime 2. B) Timeline for in vivo studies for porous and non-porous device of the inventions.

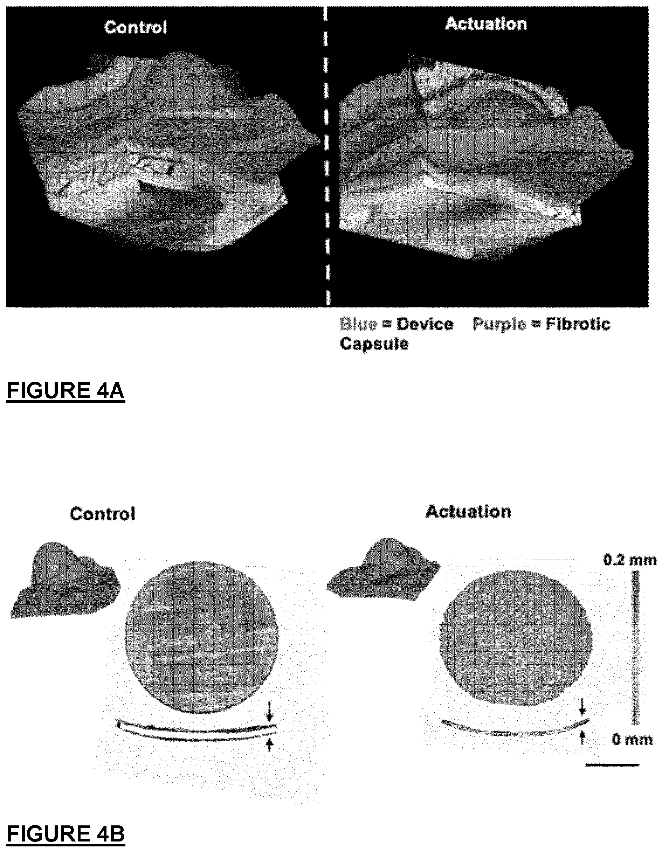

**Figure** 4: Device of the invention reduces the fibrous capsule thickness in vivo. A) MIMICs software (Materialise, Leuven) reconstruction of soft tissue stained with PMA and imaged with microCT, where the device is shown in blue and the fibrotic capsule is shown in purple. B) Thickness analysis in MIMICs where surface shell elements are shown, and thickness is calculated as the distance between them. C) Average thickness across the fibrotic capsule, n=3-6/group, data are mean $\pm$ SD, ***p<0.001. On day 15 the devices were explanted en bloc with surrounding tissue and stained with phosphomolybdic acid (PMA) to enable visualization of collagen dense tissues. Images of the device and surrounding soft tissue were acquired using microCT. MIMICs (Materialise) software was used for segmentation of the device space (Fig. 4A shown in blue) and the fibrotic capsule present on the membrane of the device (Fig. 4A shown in purple). Volumetric reconstructions of the fibrotic capsule were generated, and a thickness analysis was performed to identify mean capsule thickness. There was no significant difference between the two actuation regimes (regime 1 and 2); so we collapsed the variable and focused on analyzing the difference between control and actuated groups. A significant reduction in the mean fibrotic capsule thickness was seen when the device was actuated compared with the control (***P = 0.0005) see Fig. 4B and C.

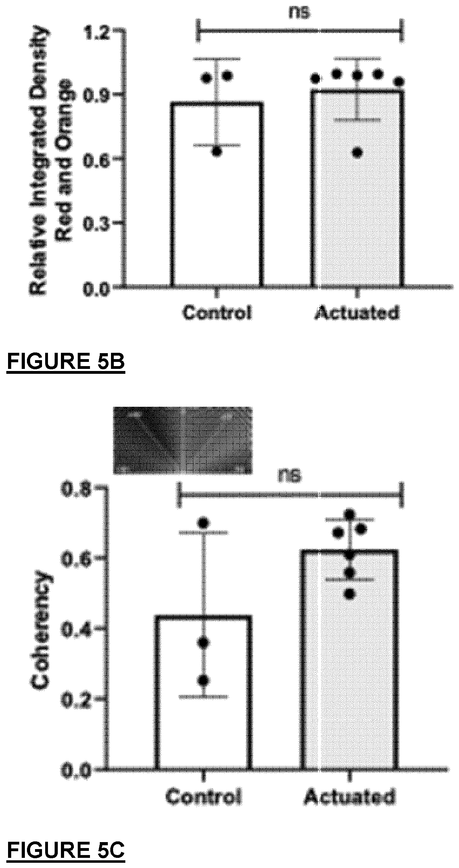

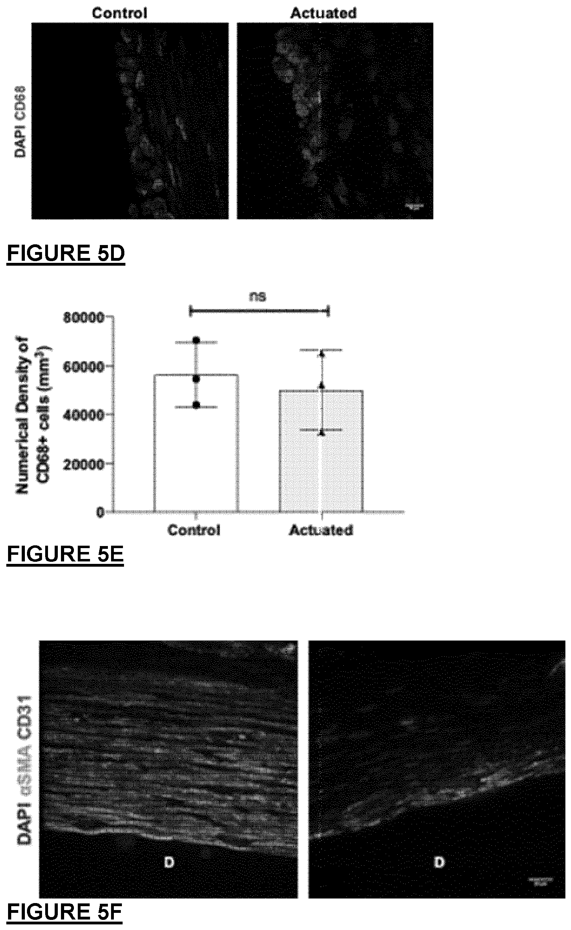

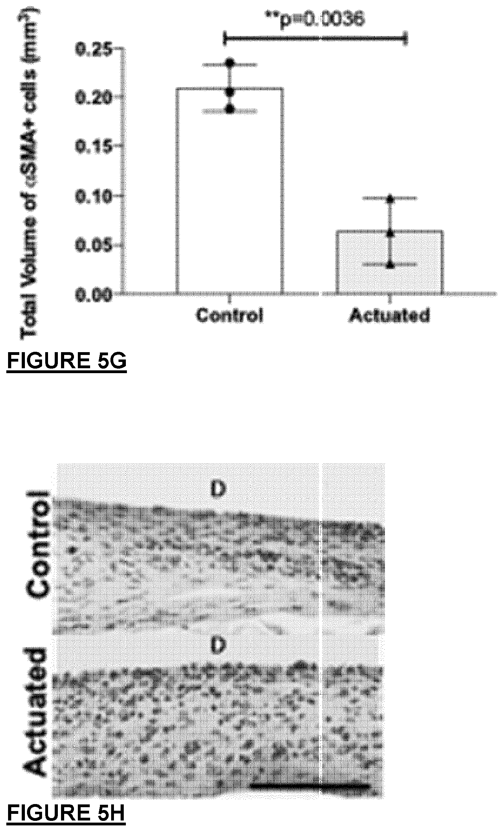

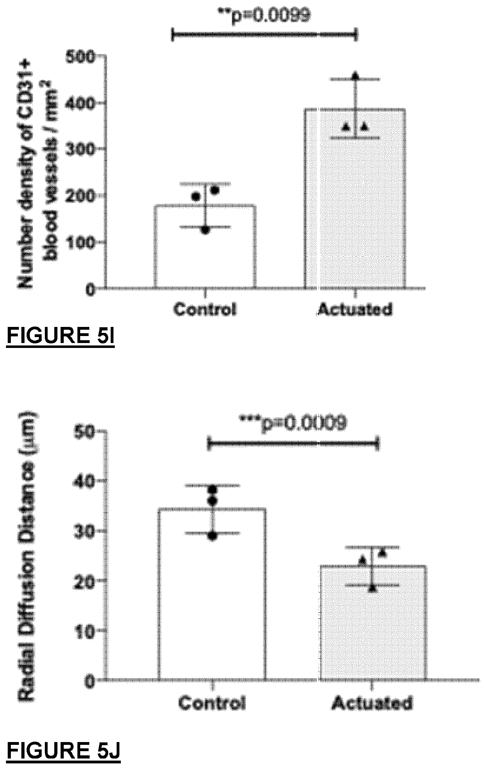

**Figure** 5: Histological analysis of the fibrous capsule. A) Relative integrated density of yellow and green fibres (signifying immature collagen) from polarized light microscopy images of the fibrous capsule in response to different treatments, with representative images shown. B) Relative integrated density of red and orange fibres (signifying mature collagen) from polarized light microscopy images of the fibrous capsule. C) Coherency of fibrous capsule based on polarized light microscopy. D) Representative immunofluorescent images of capsular tissue sections stained with CD68 (Red = CD68, Blue = Hoechst). E) Numerical density of CD68 stained macrophages in different treatment groups. F)

**Representative** immunofluorescent images of capsular tissue sections stained with $\alpha$SMA (Blue = DAPI, Green = $\alpha$SMA, Red = CD31). G) Total volume of $\alpha$SMA+ cells ($mm^3$). n=3-6/group, Data are mean $\pm$ SD, **p<0.01 To characterize the effect of actuation on the cellular constituents involved in the FBR, devices explanted on day 15 were sectioned for histological analysis. Collagen fibres are characteristically birefringent which is enhanced with picrosirius staining. Polarized light microscopy was used to assess the quality and organisation of the resulting fibrous capsule after staining. Quantification of birefringent fibers, using color threshold segmentation for mature fibers (red/orange) and immature fibers (green), yielded no statistically significant difference between groups (P > 0.05), irrespective of polarization color (Fig. 5, A and B) with an unpaired Student's t test. Quantification of the directional uniformity (coherency) of the collagen fibers showed no statistically significant difference in the fraction of fibers (P > 0.05, Fig. 5C) with an unpaired Student's t test, suggesting that actuation did not result in a compositional change in the collagen within the fibrous capsule.

Tissue sections were stained with CD68, a pan-macrophage marker, to assess the impact of actuation on the macrophage response at the tissue-device interface. Immunofluorescent images (Fig. 5D) were acquired for the control and actuated devices, and a stereological approach was used to provide a quantitative analysis of macrophage number. Numerical density was calculated (Fig. 5E) and referred to the number of, in this case, macrophages within a unit volume of tissue. A paired Student's t test was carried out with no statistically significant difference observed between groups (P = 0.6963), suggesting that actuation did not influence macrophage number at the device surface. Immunofluorescent staining was also performed on the tissue sections for $\alpha$-smooth muscle actin ($\alpha$SMA) to assess for the presence of myofibroblasts around the control and actuated device (Fig. 5F). An unpaired Student's t test showed the total volume of $\alpha$SMA-positive ($\alpha$SMA+) cells to be significantly lower with actuation compared present around the control and actuated devices. Number per area and radial diffusion distance were all calculated using an unbiased counting frame. An unpaired Student's t test showed a significant increase in the number of CD31+ blood vessels (**P = 0.0099, Fig. 5I) and a significant decrease in radial diffusion distance for the actuated device compared with the non-actuated controls (***P = 0.0009, Fig. 5J). This indicated an increased vascularity of the capsule after actuation.

**Figure** 6: Arrays of non-porous device of the invention reservoirs to reduce complications associated with implantable devices. Circular arrays are encapsulated around a breast implant, and a linear array is integrated into a cylindrical structure, ultimately envisioned for pacemaker leads. Device of the invention arrays are shown in colour, with each colour representing an individual actuation line. Incorporating arrays of non-porous device of the invention reservoirs into medical devices that suffer from an adverse host response helps to reduce these effects. We demonstrate physical embodiments of circular and linear arrays of device of the invention, with reservoirs in series and in parallel, by encapsulating multiple reservoirs (manufactured from thermoplastic urethane or silicone - shown in Fig. 6), and their actuation lines in a silicone sheet around a breast implant, and tubular structure that is ultimately envisioned for pacemaker leads or indwelling catheter lines (Fig. 6).

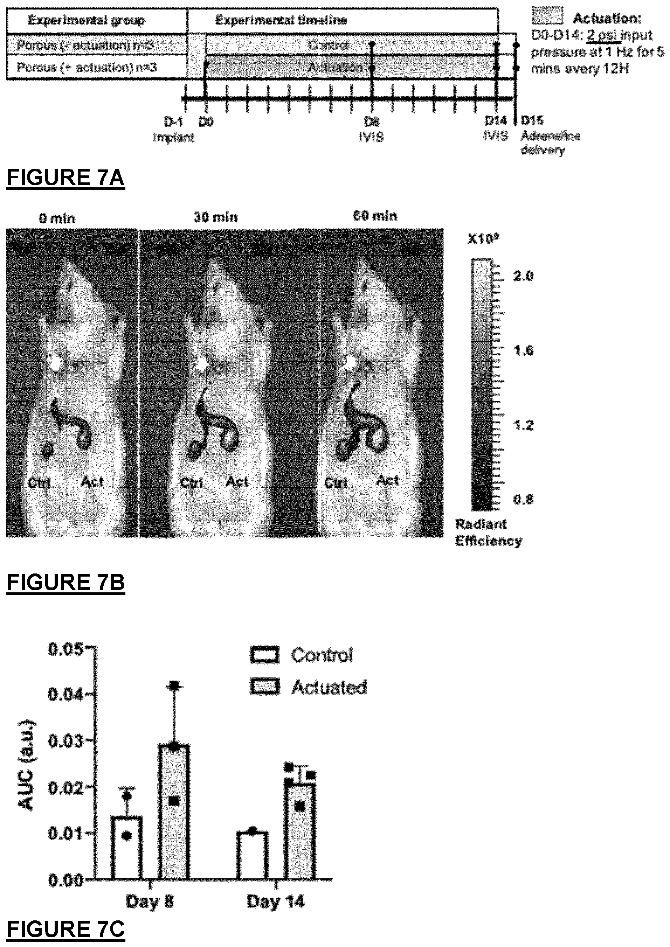

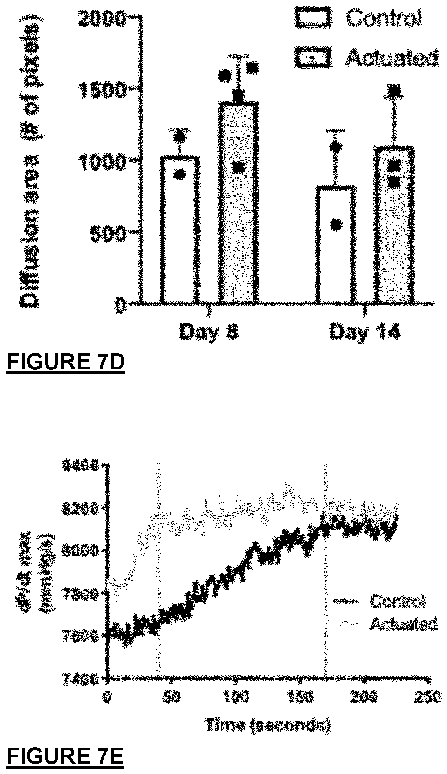

**Figure 7:** Enhanced pharmacokinetics through a porous device with actuation. (A) Timeline for in vivo studies for porous device. (B) Images from the IVIS at day 14 at 0, 30, and 60 min showing the implanted actuation and control groups. (C) Area under the fluorescence curve at days 8 and 14 for actuated and control groups, n = 2 to 4 per group per time point, data are means $\pm$ SD. (D) Diffusion area at days 8 and 14 for actuated and control groups, n = 3 to 4 per group per time point, data are means $\pm$ SD. (E) dP/dt max, a measure of ventricle contractility for the actuated and control devices in a representative animal. The vertical dashed lines show time to therapeutic effect for the control and actuated groups. (F) Average dP/dt max per cycle for the first 40 s (the time to therapeutic effect for the actuated group). Ctrl, control; Act, act; AUC, area under the curve; a.u., arbitrary units. To investigate the transport of therapy through the resulting fibrous capsule, we injected a therapy analog Genhance (PerkinElmer) through the therapy delivery catheter of a control and actuated porous device and subsequently through the porous membrane to the surrounding tissue at days 8 and 14 (see Fig 7A for timeline). Fluorescent in vivo imaging was conducted for 1 hour after delivery of drug analog (Fig. 7B). Fluorescence (radiant efficiency) was plotted for each animal for 1 hour after delivery and for the control and actuated device, and the area under the radiant efficiency curve was calculated (Fig. 7C). There was a decrease in the amount of drug analog delivered to an area of tissue surrounding the implant over time, as expected due to the attenuation of drug diffusion caused by the FBR (Fig. 7C). However, the actuated devices delivered more drug analog compared with the control at each time point (37 and 16% mean improvement at days 8 and 14, respectively; Fig. 7C). The area of diffusion was also quantified with the actuated groups showing a larger spatial area of diffusion [in a two-dimensional (2D) plane] than the control after 1 hour at days 8 and 14 (Fig. 7D). As a model therapy to visualize rapid drug delivery, epinephrine was delivered through the control and actuated devices (n = 1). Using a pressure-volume catheter, we measured the maximum rate of change of the interventricular pressure (dP/dt max is a measure of the contractility of the ventricle) immediately after delivery of the drug. The time to therapeutic effect is defined as the time when the dP/dt max leveled off and stopped increasing. This time is indicated by dashed lines for a control and actuated device in the same animal (Fig. 6E) and is lower (~40 s) for the actuated device compared with the control (~170 s). The cyclical average dp/dt max in the first 40 s after delivery was significantly higher (P < 0.0001) in the actuation group compared with the control (Fig. 7F), acting as a clinically responsive model to demonstrate enhanced delivery after the actuation regime.

**Figure** 8: Implantable actuation system: A) Schematic of the piezo-electric pump and the closed loop cycle to device of the invention, a resistive component, and a capacitor or compliant component. B) Test system with external electropneumatic control box. C) Measured pulsatile force generation from implantable pump compared to measured average peak force with external control box.

**Figure** 9: Test set-up for force characterization and porous vs non-porous computational models. A) Acrylic holder for supporting reservoir and ensuring that lower functional membrane is in contact with upper cross-head. B) Test set-up in mechanical tester. C) Strain energy density function for Ogden model. D) Light microscopy of laser-cut porous membrane. Pore diameters were 10 micron with spacing of 200 microns. E) Boundary conditions applied to the finite element model. F) In-plane strain and G) Mises stress for the porous vs non-porous device of the inventions showing relatively small differences.

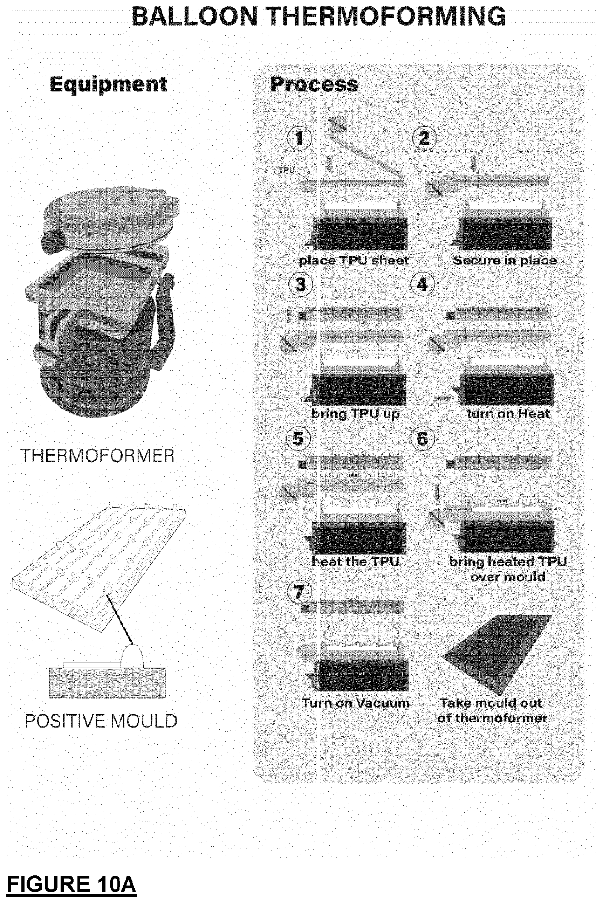

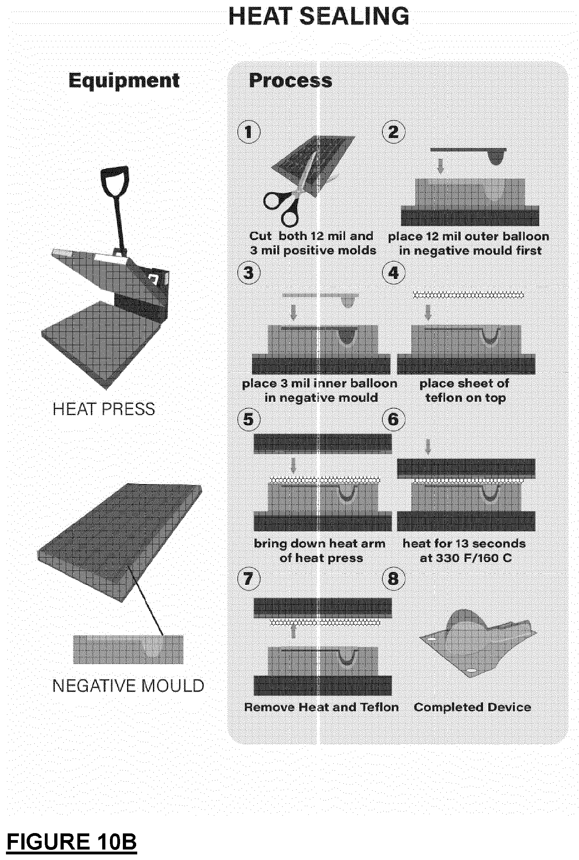

**Figure 10:** Device manufacture procedure which involves two step - balloon thermoforming and heat sealing. **Balloon Thermoforming** (Fig. 10A): 1) Thermoplastic urethane (TPU) sheets (0.07 mm and 0.3 mm) were mounted separately in a thermal former with a vacuum platform and a positive 3D printed mould was placed on the platform, 2) The TPU sheet was secured in place, 3) The TPU was brought close to the heating element, 4) the heat was turned on, 5) the TPU was heated until it sags in the centre, 6) the platform was lowered over the positive mould, 6) the vacuum was applied to form the sheet over the positive mould, 8) the formed TPU was removed from the positive mould. **Heat sealing** (Fig. 10B): 1) The formed TPU was cut out, 2) 0.3 mm TPU was placed in a 3D printed negative mould, 3) 0.07 mm TPU was placed in a 3D printed negative mould on top of the 0.3 mm TPU, 4) A teflon strip was inserted into the groove of the negative mould between relevant layers of TPU to keep the catheter channels open during the following heat-sealing 5) a 0.07 mm TPU membrane (porous or non-porous) was placed on top and a layer of Teflon was played on top to protect the assembly, 5) the arm of the heat press was brought down on top of the mould, 6) the assembly was heat sealed with a heat transfer machine, 7) the heat arm and Teflon was removed, 8) device reservoir was complete. Catheter tubing was inserted into relevant channels and heat shrink tubing was used to seal the devices.

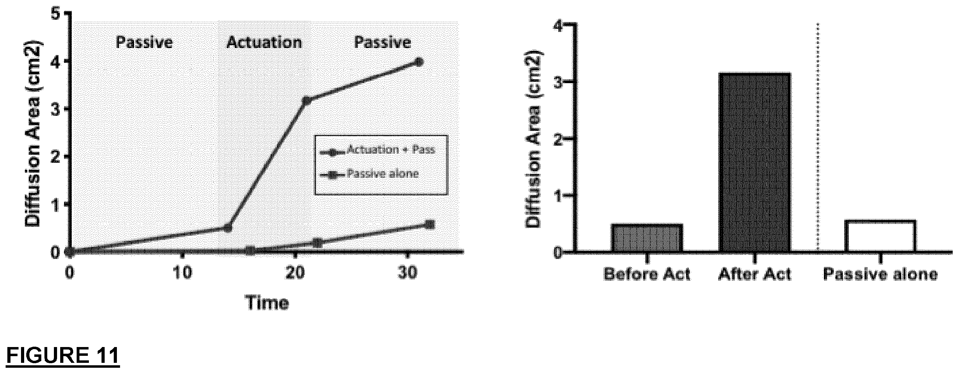

**Figure 11:** 21 day rodent model comparing passive and actuation release following chronic implantation of the porous device. Graphs depict drug diffusion area to surrounding tissues of drug analogue Genhance 750 in a passive and actuated device

Detailed Description of the Invention

The invention relates to an implantable medical device having a soft-tissue interfacing surface comprising one or more capsules actuatable to reduce fibrotic encapsulation of the medical device. In any embodiment the capsules have a soft-tissue interfacing deflectable membrane configured for cyclic deflection upon actuation of the capsule. Cyclical deflection of the membrane is configured to alter the biomechanics of the soft-tissue interface by altering one or more of displacement, force, strain, fluid flow and cellular activity in peri-implant tissue at the soft tissue interface, resulting in inhibition or reduction of fibrotic encapsulation of the medical device. In any embodiment the frequency of cyclical deflection may be 0.1 to 10Hz. In any embodiment the actuation pressure (e.g. the pressure applied by the deflection of the membrane, or the pressure applied to the capsule or a working chamber of the capsule) may be 0.5 to 20 PSI or 0.5 to 10 PSI. In any embodiment the waveform of the cyclical deflection may be sine, square, triangular or sawtooth. In any embodiment the implantable medical device is of the type that performs a primary function in the body (e.g a tissue implant, sensor, catheter, cannula, dilator) and that is susceptible to fibrotic encapsulation. In any embodiment the primary function of the medical device is generally independent of the actuatable capsule (whose function is to reduce fibrotic encapsulation and in one embodiment release drug). In any embodiment, the primary function is not drug delivery or drug infusion. In any embodiment the deflectable membrane is porous or configured or configured to become porous upon actuation or deflection. This allows the capsule also release drug into the peri-implant space, where the cyclical deflection of the membrane can elicit rapid movement/propulsion of the drug into surrounding body tissue or, when the device is subject to fibrotic encapsulation, into surrounding tissue through the fibrotic capsule. Thus, in any embodiment, this rapid movement can be used to overcome physical barriers such as fibrous capsule formation around the device/implant. In any embodiment the capsule may incorporate a spacer configured to space the soft-tissue interfacing deflectable membrane form the soft-tissue and provide a gap where drug from the capsule can fill. The spacer may be annular.

The implantable medical device may include an actuator for the deflectable membrane. The implantable medical device may include a controller (which may be programmable) for the actuator configured to modify the output parameter(s) of the actuator. Output parameters may include frequency, pressure, amplitude, waveform and ramp speed. The capsule is soft (e.g not rigid) and generally has an Elastic Modulus of less than 1 GPa (for example 10 KPa to 1 GPa, 10 KPa to 1000 KPa, 1 mPa to 1000 MPa, 1 MPa to 500 MPa, 1 MPa to 200 MPa, 10 MPa to 50 MPa, 10 MPa to 20 MPa). The deflectable membrane is soft and flexible to allow cyclical deflection. The actuatable capsule may be fluidically actuatable (e.g. pneumatic or hydraulic actuation). The actuatable capsule may comprise a therapeutic chamber and a working (actuation) chamber separated by a deflectable membrane, where the therapeutic chamber comprises the soft-tissue interfacing deflectable membrane. Thus, when the actuation chamber is fluidically actuated (e.g. inflated and deflated cyclically), this causes the therapeutic chamber to inflate and deflate causing the soft-tissue interfacing deflectable membrane to deflect outwardly and inwardly. This embodiment provides for greater control of actuation. In one embodiment, the capsule may comprise one chamber configured for cyclical inflation and deflation and consequent cyclical deflection of the soft-tissue interfacing deflectable membrane. In one embodiment, the capsule is configured for electrical or ultrasound actuation to cause cyclical deflection of the soft-tissue interfacing membrane. In one embodiment, the implantable medical device is not an implantable drug infusion device and in particular not an implantable drug infusion device having a pressure counterbalancing mechanism. In any embodiment, the device of the invention includes a passive drug infusion capsule. In one embodiment, the passive drug infusion capsule may be disposed adjacent to an actuatable capsule.

The implantable medical device may be any implantable device that is subject to the problem of fibrotic encapsulation. Examples include tissue implants (e.g. breast implant), indwelling catheters, dilators or introducers, pacemakers, biosensors, a prosthetic joints, valves, shunts, electrical leads or batteries, drug or cell delivery devices, vascular grafts, hernia meshes, plastic surgery augmentation devices, sensors, and probes, pumps, and contractile organ augmentation devices.

Definitions and general preferences

Where used herein and unless specifically indicated otherwise, the following terms are intended to have the following meanings in addition to any broader (or narrower) meanings the terms might enjoy in the art:

Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae,* which includes horses, donkeys, asses, kiang and zebra.

"Implantable medical device" should be understood to mean an indwelling medical device that depends on an interface with native tissue. Examples include neural probes [1, 2], indwelling catheters [3], mammary implants [4], pacemakers [5], glucose biosensors [6-8] and drug and cell delivery devices [6, 9-12]. Such devices are often subject to a collection of biological host responses such as fibrosis, bacterial biofilm formation and inflammation, which can impair functionality. At present, device failure is expected and inevitable, and the costs, inconvenience and morbidity that it can impose on patients are largely accepted. The present invention proposes modifying the implantable medical device to reduce FBR by incorporating one or more capsules on a soft tissue interfacing surface of the device. The capsules include a soft tissue interfacing deflectable membrane configured for deflection (typically cyclical deflection) when the capsule is actuated, which alters fluid flow and strain in the soft tissue interface reducing FBR. In one embodiment, the implantable medical device is not an implantable drug infusion device and in particular not an implantable drug infusion device having a pressure counterbalancing mechanism and/or expansion chamber. In one embodiment, the implantable medical device has a primary function that is independent of the cyclical deflection of the soft-tissue interfacing deflectable membrane. The term "primary function" as applied to the medical device means the main purpose of the device, for example the primary purpose of a tissue implant is to replace tissue removed (e.g. a breast implant), the primary function of a pacemaker is to control the electrical activity of the heart, the primary function of a catheter may be to drain fluid from a body lumen (e.g. bladder or pleural cavity), the primary function of a glucose sensor is to sense glucose levels in a body fluid).

"Soft tissue interfacing surface" refers to a part of the surface of the implantable medical device that interfaces with soft tissue in a subject. This surface may form all or part of the surface of the medical device. It will be appreciated that the invention may also be employed in devices that interface with hard tissue, such as bone.

"Actuatable capsule" refers to a soft capsule having a soft tissue interfacing membrane configured for deflection when the capsule is actuated. Actuation of the capsule may involve inflation of the capsule, for example fluidic (pneumatic or hydraulic) inflation. Alternative methods of mechanical actuation of the capsule are envisaged. For example, ultrasound may be employed to cause deflection of the soft tissue interfacing deflectable membrane. The capsule may include an ultrasound emitted configured to generate shock waved in the fluid in the capsule by cavitation, leading to deflection of the deflectable membrane. Alternatively, an external ultrasound emitter may be employed to actuate the capsule. In another embodiment, the part of the capsule (for example the soft tissue interfacing deflectable membrane) may comprise an actuating material (for example a charged material) that is configured to actuate the capsule when electrically stimulated. The actuating material may be a piezoelectric material. The deflectable membrane may comprise actuating material. The capsule may be dome shaped, with the soft tissue interfacing membrane typically forming a base of the capsule. The capsule may comprise two chambers, an actuation chamber and a therapeutic chamber, separated by a deflecting separating membrane or barrier. The actuation chamber is generally disposed in the capsule adjacent the medical device, and the therapeutic chamber is generally disposed adjacent tissue. The actuation chamber is generally larger than the therapeutic chamber, with the therapeutic chamber disposed within the actuation chamber. The capsule and/or membranes of the capsule are generally formed by a soft polymeric material, for example any thermoplastic or mouldable material such as a polyether film (i.e. TPU polyether). In one embodiment, the soft tissue interfacing membrane is porous to allow the second fluid in the therapeutic chamber pass through the membrane into the surrounding tissue. The size of the pores is variable and depends on the size of the active agent being delivered, and in one embodiment is sized between 5-15 microns.

"Millisized" as applied to an actuatable capsule refers to a capsule having a maximum diameter and height in the mm range, for example of not greater than 30mm (i.e. 1-30 mm), for example a diameter and height of about 5-20 mm or preferably about 10-15 mm.

It will be appreciated that other sizes of capsule may be employed, for example capsules having a height and width in the micro range or in the cm range.

"Fluid" refers to a gas or a liquid, typically a gas which is preferably air or saline. The first and second fluids may be the same or different. Generally, the therapeutic chamber contains a gas.

"Array" refers to an arrangement of a plurality of capsules on the soft tissue interfacing surface of the implantable medical device. The array may be an ordered array. In one embodiment, the array is configured such that adjacent capsules are separated by a distance of 1-5 cm.

"Skin" or "retro-fit skin" refers to a cover or sheath member comprising one or more capsules and configured for adhering to all or part of a surface of the implantable medical device to form all or part of the soft-tissue interfacing surface of the implantable medical device. The skin can be attached to the medical device during manufacture, or retro-fitted to the device after manufacture and prior to use. The skin can be provided separately to the medical device. The skin can be shaped to conform to a surface of the medical device, for example it can be shaped to conform to a breast implant, or may be cylindrical or a sheath to conform to an indwelling catheter. Generally, the soft tissue interfacing membrane of the capsules are flush with surface of the skin. The skin is generally soft and pliable, and may be formed from a soft polymer such as silicone.

"Active agent" refers generally to an agent or component that has a pharmaceutical or biological effect in a mammal, or diagnostically active agent such as an imaging dye or radioligand or antibody. Examples include glucose, insulin, drugs, for example, a drug to relieve pain such as non-steroidal anti-inflammatory drug (such as Ibuprofen, Ketoprofen or Naproxen), aspirin, acetaminophen, codeine, hydrocodone, an anti-inflammatory agent such as a steroidal anti-inflammatory agent, an anti-depressant, an anti-histamine, or an analgesic, polysaccharides, proteins, peptides, polypeptides, antigens, antibody (monoclonal or polyclonal), antibody fragments (for example an Fc region, a Fab region, a single domain antibody such as a nanobody or VHV fragment), a conjugate of an antibody (or antibody fragment) and a binding partner such as a protein or peptide, a nucleic acid (including genes, gene constructs, DNA sequence, RNA sequence, miRNA, shRNA, siRNA, anti-sense nucleic acid), cellular products such as growth factors (i.e. EGF, HGF, IGF-1, IGF-2, FGF, GDNF, TGF-alpha, TGF-beta, TNF-alpha, VEGF, PDGF and an interleukin).

"Fluidic resistor" refers to a part of the second conduit that resist the flow of fluid back to the pump. The resistor may comprise a section of conduit which is narrowed compared with the first conduit. Thus, when the pump is actuated and fluid is pumped into the actuation chamber, the resistance to flow in the outlet (second) conduit helps cause an increase in pressure in the actuation chamber. The outlet (second) conduit also includes a "Fluidic capacitor" downstream of the fluidic resistor. The purpose of the capacitor is to allow for pressure release in the fluid when the pump is off, and it generally comprises an inflatable chamber that receives fluid and expands to accommodate the fluid pending actuation of the pump.

"Fibrotic encapsulation": Once a device is implanted a complex series of events is initiated to protect the host from the 'foreign body'. Shortly after implantation, fibrinogen and other proteins bind to the surface of the device/foreign body, macrophages then bind and, over time, become multinucleated giant cells releasing inflammatory cytokines. In response to these signals, quiescent fibroblasts are transformed into myofibroblasts, which synthesize procollagen. The procollagen becomes crosslinked and this mature crosslinked collagen and other extracellular matrix proteins contribute to the formation of a dense fibrous capsule that becomes impermeable or hypo-permeable to many compounds.

"Spacer" refers to an element configured to space the soft-tissue interfacing deflectable membrane from the soft tissue during use. The spacer may be part of the deflectable membrane or a separate element. The spacer functions to define a volume between the deflectable membrane and the soft tissue that in use provides a space for drug to enter after passing through the deflectable porous membrane. The spacer may be annular.

"Actuator" and "controller": The implantable medical device of the invention may include an actuator to cause cyclical deflection of the soft-tissue interfacing deflectable membrane. The implantable medical device may include a controller (which may be programmable) for the actuator configured to modify the output parameter(s) of the actuator. The actuator may be a fluidic (pneumatic or hydraulic) actuator configured to effect cyclical deflection of the soft-tissue interfacing deflectable membrane by altering the fluidic parameters of the capsule (or actuating chamber of the capsule). Output parameters may include frequency, amplitude or wave form of the periodic force applied to the capsule by the actuator. The actuator may be configured to actuate the capsule at a ramp speed of less than 60, 50, 40, 30, 20, 10 or 5 seconds. The ramp speed means the time it takes for a target cyclical deflection parameter (i.e. 1 Hz) to be achieved.

Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

Materials and Methods

*device of the invention fabrication*

The device manufacture technique for the individual reservoirs is shown in Fig. 10. Briefly, 3 mil (0.075 mm) and 12 mil (0.3 mm) thick thermoplastic urethane TPU sheets (HTM-8001-M and HTM-1001 polyether TPU film, American Polyfilm, Inc) were formed into hemispherical shapes, with a height of 3.9 mm and a diameter of 3.5 mm, using a vacuum thermal former (Yescom Dental Vacuum Former, Generic). 3 mil (0,075 mm) thick TPU hemispheres were placed inside the 12 mil (0,3 mm) thick TPU hemispheres and 7 cm of 3 Fr thermoplastic polyurethane catheter tubing (Micro-Renathane MRE037 0.037" x 0.023, Braintree scientific, where one inch is approximately 25,4 mm) was bonded to this assembly using a heat transfer machine (Heat Transfer Machine QXAi, Powerpress). This forms the actuation reservoir, which was used for actuating the system with pneumatic pressure via the catheter. A 3 mil (0,075 mm) thick TPU membrane and a second 9 cm 3 Fr (1 mm) thermoplastic polyurethane catheter was then bonded onto this assembly to form the therapy reservoir which can be used to deliver therapeutics prior to or post implantation via the catheter. This reservoir interfaces with soft tissue via the TPU membrane which can either be nonporous or porous (Fig. 1B) (10 $\mu$m pores, National Centre for Laser Applications, National University of Ireland Galway).

*Implantable system*

A fully implantable stainless steel piezoelectric pump (Dolomite 3200311) was used to demonstrate cyclic actuation of device of the invention. The device was placed in series with a resistive component consisting of narrower diameter tubing (0.2 mm ID, 0.5 mm OD, Polyethylene tubing, SAI Infusions) and a capacitor consisting of a TPE reservoir (2 mm by 1.5 mm, Stretchlon 200, FibreGlast) (Figure 8). Turning the pump on and off generated cyclic actuation of device of the invention. While the pump was on, the resistive component caused filling of the device reservoir. While the pump was off, fluid flowed from the reservoir to the capacitor and energy was dissipated. Using this circuit, we were able to recapitulate the average peak force achieved with the external control box (Figure 8B). The pump was operated with the pump control board (Dolomite 3200313) and the Mitos Flow Control Centre (FCC) software Figure 8B). The output voltage was +/-80 V, the pulse width was +/- 20 $\mu$s, and the frequency was 350 Hz.

*Experimental characterization of the device of the invention*

ASTM D638 uniaxial tensile tests were performed on a ZwickRoell material testing machine with laser cut TPU specimens. Using the material evaluation feature in Abaqus 2018 (Dassault Systèmes, Vélizy-Villacoublay, France), four hyperelastic constitutive models (Neo-Hookean, Yeoh, Mooney-Rivlin and Ogden 3rd order) were evaluated. As shown in Fig. 2b, the Ogden 3rd order model had excellent curve fitting performance and therefore was used for the model. The strain energy density function is shown in Fig. 10C. The actuation force of the device was measured using an Instron 5944 material testing machine. The set-up is shown in Fig. 10. An acrylic test-rig was laser cut to allow the lower membrane of the device to contact the upper compression plate for force measurement. The thickness of the acrylic piece (10 mm) and the diameter of the hollow cylinder (5 mm) were designed to be larger than the height and the diameter of the outer balloon allowing unconstrained deformation of the reservoir. The distance between the compression plates was fixed at the thickness of the acrylic holder. The force was measured using a 50 N load cell. The test the effect of fatigue on the devices the in vivo actuation regimes (Fig. 2 which corresponds to 100,000 cycles) were applied to device of the invention in vitro and burst pressure testing was carried out (n=6/group). A piezoelectric pressure sensor (AMS5812, Analog Microelectronics Ltd) with an Arduino UNO interface was connected to the device and syringe via a T-valve. A syringe pump was used to inflate the devices to failure and burst pressure was recorded.

*Computational modelling*

All simulations were conducted in the commercially available software, Abaqus 2018 (Dassault Systèmes, Vélizy-Villacoublay, France). Since the reservoir is comprised of thin thermoplastic urethane sheets, shell structural analysis was chosen for the simulation. The device was modelled as a 3D surface geometry, which contains the outer, middle and lower membrane, as shown in Fig.9. The appropriate thickness of each shell was assigned. All parts were meshed with 2603 four-node shell elements (S4R). Dirichlet boundary condition were applied to the protruding edge of the lower membrane and all other surfaces were allowed to deform freely. A 2 psi ramp loading was applied to the internal surface of the inner and outer balloons for a duration of 500 ms. These boundary conditions were representative of the in vivo scenario when the reservoir was sutured to the muscular tissue by the edge of the lower membrane and actuated with a cyclic pressure with magnitude of 2 psi and 1 Hz.

We also created a fluid structure interaction model using the smooth particle hydrodynamics (SPH) technique to study the effect of actuation on surrounding fluid flow. A $20\times20\times5$ mm3 region was created and filled with 8000 particles. The particles were assigned with a density of 9.96e-7 kg/mm$^3$, bulk modulus of 20.94 MPa and dynamic viscosity of 3.56e-8 MPa.s. Contact pairs were initiated between the shell structure and the particles. Loading and boundary conditions of the shell were the same as the structural analysis.

*In vivo studies*

Animal procedures were reviewed and approved according to ethical regulations by the Institutional Animal Care and Use Committees at Massachusetts Institute of Technology. Female Sprague Dawley rats (225-300 g) were anaesthetized using isoflurane (1-3% isoflurane in oxygen). Animals were treated with a single dose of sustained release buprenorphine (Bup-SR) at 1 mg/kg subcutaneously to control pain. 2 device of the invention reservoirs (porous or non-porous) were implanted subcutaneously in the rat, the device implantation procedure is shown in Fig. 3A. Briefly, the hair on the back of the rat was removed and surgical sites were prepared with 3 washes of Betadine and 70% ethanol. Devices were sterilized using ethylene oxide prior to implantation. An anterior incision was made at the base of the neck for the and 2 posterior incisions were made 9 cm from the original incision along the back of the rat, 1 cm lateral of the spine. A blunt dissection was made at all incisions and a forceps was used to tunnel subcutaneously from the anterior to the posterior sites. A vascular access button, or self-sealing subcutaneous port (VAB95BS-MRI, VABM2B/22R22, VABR4B/22 Instech Laboratories), was connected to the dorsal end of the catheter of each device of the invention reservoir. The port was placed in position at the base of the neck and the devices tunneled posteriorly into position. The port was secured to the underlying fascia using at least one interrupted suture (5-0 monofilament). Each device of the invention reservoir was secured to the underlying fascia with one suture at either side (7-0 monofilament). The skin was closed with interrupted sutures (5-0 monofilament) and the animal was allowed to recover on a heated pad. 3 mL of warm saline were administered subcutaneously. The following day, one of the two implanted device of the invention reservoirs was actuated at controlled input pressures of 1 PSI (Regime 1) or 2 PSI (Regime 2) at 1 Hz every 12 hours for 14 days (Fig. 3b) and compared to the non-actuated control reservoir for analysis. On day 15, animals were sacrificed by CO2. Following sacrifice, each device and the immediate surrounding tissue were extracted. Tissues were fixed for 24 h using 10% Formalin (pH 7.4). The tissue was then washed in 0.2 M phosphate-buffered saline with a final wash in 70% ethanol.

*IVIS imaging*

On days 3, 8 and 14 post-implantation, diffusion of a fluorescent imaging agent (Genhance 750 Fluorescent Imaging Agent, NEV10118, Perkin Elmer) out of the porous device of the invention reservoirs was evaluated using an IVIS Spectrum-bioluminescent and fluorescent imaging system (Perkin Elmer). Images were acquired using a 745 nm/800nm excitation-emission filter pair. Prior to injection of agent, the line was cleared by pulling a vacuum with a syringe connected to the subcutaneous port. The skin above the device of the invention reservoir area was shaved and marked to aid with region of interest (ROI) placement for quantitative analysis. After a background image was acquired, 35 µL of fluorescent agent were injected through the subcutaneous port either manually or with a syringe-pump (150 µL/min, Harvard Apparatus). Images were acquired for up to 1 hour following injection ~ every 3 min. An ROI (28.24x28.24 pixels) was placed at the centre of each device of the invention reservoir in every image and total radiant efficiency was measured using the Living Image 3.2 software. Total radiant efficiency measured immediately after injection was subtracted from the subsequent measurements to only account for the change in radiant efficiency due to diffusion over an hour.

*Functional measurements following epinephrine injection*

To illustrate how actuation can improve the time to functional effect after small-molecule delivery, 35 uL of epinephrine (1mg/ml) were delivered through the system while recording blood pressure with an apically inserted pressure-volume catheter (Millar) as previously described [13].

*microCT, histological and immunohistochemical analysis*

For microCT analysis, fixed tissue samples were transferred to a 2.5% PMA solution in 70% ethanol for 7 days, then washed and stored in fresh 70% ethanol. MicroCT images were captured using a µCT 100 scanner (Scanco) at 70 kVp and 85 µA with a 0.5mm Aluminum filter. microCT dicom files were segmented using Mimics Research 18.0.0.525 software as described in Supplementary Material.

Fixed tissue samples (n=3) were transected in half, orientated and embedded in paraffin wax blocks for histological and immunohistochemical analysis. Sections of 5 µm were cut, deparaffinised in xylene, and rehydrated through a series of graded alcohols. For histological fibrotic capsule quality analysis, sections were stained with 0.1% Picrosirius red/fast green solution (1:1) using an automated stainer Leica ST5010 Autostainer XL. Slides were imaged using Ocularâ2.0 Imaging Software on an Olympus BX41 Microscope with Olympus U-AN360P analyser under a 20x objective lens. Quantification of the collagen content was performed using a previously reported technique [17] and described in Supplementary Material. For immunohistochemical analysis, primary antibodies of CD31 (ab28364, Abcam) (1:200), CD68 (MCA341, Bio Rad) (1:300) and α-SMA (ab5694, Abcam) (1:100) were incubated for 1 hour at 37 °C. Secondary antibodies of Alexa Fluor® 594 goat anti-mouse IgG (ThermoFisher Scientific) (1:200), Alexa Fluor® 594 goat anti-rabbit IgG (ThermoFisher Scientific) (1:200) and Alexa Fluor® 488 goat anti-mouse IgG (ThermoFisher Scientific) (1:200) were incubated for 60 min at room temperature, respectively. Sections were stained with Hoechst and coverslipped using fluoromount. Immunofluorescence-stained slides were observed using a spinning disc inverted confocal microscope (Yokagawa CSU22) combined with Andor iQ 2.3 software. Analysis of Macrophages: 20 random fields of view were acquired from 8 sections using confocal microscopy. Using stereological methods an unbiased estimation of area fraction and numerical density (Nv) was calculated [14-16]. Numerical density refers to the number of, in this case macrophages, within a unit volume of tissue. Analysis of Myofibroblasts: 20 random fields of view were acquired from 8 sections using confocal microscopy. Area Fraction was estimated using Image J (Fiji verson 2.0.0) software. This was done by way of auto thresholding using the otsu filter which eliminates autoflouresence and calculates the area fraction of tissue occupied by αSMA+ cells. The area fraction was then multiplied by the average volume of c in both control and regime groups to provide an estimate of the total volume of αSMA+ cells to assess whether the presence of myfibroblasts were perturbed by actuation.

*Statistical Analysis*

GraphPad Prism (8.1.0) or Stata version 16.0 was used for statistical analysis. Nonparametric tests were sometimes performed for n ≤ 3 per group, in part because the sample was too small to test whether the endpoint was normally distributed. However, the t test is fairly robust to departures from normality, and in many circumstances, it is more powerful than corresponding nonparametric tests. Thus, we conducted t tests (or generalizations of t tests, as we explained) to compare endpoint measurements between the control and actuated samples. Where possible, when each animal had both a control and an actuated device, we used paired t tests. For endpoints for which some animals had data only under control or only under actuated conditions, we fitted linear regression models in a generalized estimating equations (GEE) framework, with robust standard errors and an independent correlation structure. The GEE model can be thought of as being similar to the paired t test while allowing an unbalanced design and accounting for the correlated data caused by having control and actuated measurements in the same animals. To maximize study sensitivity, we considered the primary analysis to have only two comparison groups; i.e., we collapsed data from regimes 1 and 2 into one treated group (actuation). We consider the comparison of measurements between two regimes (e.g., two different pressure levels) as exploratory; i.e., we do not consider the P value a dichotomous significance test. For burst pressure and osmolarity analyses, there were more than two groups. First, normality was tested with a Shapiro-Wilk test; if the data were normally distributed, we used a one-way or two-way ANOVA with Tukey's post hoc adjustment for multiple comparisons. If the data were not normally distributed, a Kruskal-Wallis test was used with Dunn's post hoc adjustment for multiple comparisons. Statistical significance was accepted when P < 0.05.

*MIMICS segmentation and quantification of fibrous capsule thickness*

Dicoms were opened and cropped between suture points on the device. A green mask was added to threshold the field of view. It was then cleared and edited using multiple slice editor to segment the fibrous capsule manually in the sagittal view. The fibrous capsule was mapped every five segments for the entirety of the CT dicom. The fibrous capsule was visually identified by a uniform change in signal intensity at the muscle border. After approximately fifty segments were mapped, interpolation was used to account for gaps between segments. Following completion of the green mask, an additional yellow mask was applied to the dicom files to threshold the black and remove background. A boolean operator was applied (green mask-yellow mask) and subsequently created a third mask (cyan mask). The cyan mask was then edited using multiple slice editor using axial view to ensure consistency between different views. When fibrous capsule was accurately complete in the cyan mask, it was edited in 3D. A circle (2.4 - 2.6 mm diameter) was dropped in center of fibrous capsule. The 3D file was exported as a STL for further analysis using 3-matic Research 10.0.0.212 software. Fix wizard was applied to the files. Smoothing factor of 0.8 was applied. Auto-remesh was then applied. A wall thickness analysis was generated and data was analysed.

*Quantification of fibrous capsule quality*

Fibrotic capsule quality was assessed using picrosirius red and stained in 0.1% fast green (pH 7, Fast Green FCF; Sigma Aldrich) and 0.1% Sirius red in saturated picric acid (picrosirius red stain), both in the same solution at a 1:1 ratio for 1 hour according to previously established protocols [17]. Following this, the sections were rapidly dehydrated through graded ethanol washes (70-100% v/v). Staining was performed using a Leica ST5010 Autostainer XL (Leica Biosystems; Wetzlar, Germany). The slides were then mounted using DPX mountant (Sigma Aldrich) and left to dry horizontally for five hours. Polarised light micrographs were captured using an Olympus BX4 polarised light microscope (Mason Technology Ltd. Dublin, Ireland) at 20x magnification. The polarising lenses were positioned on the light path before the sample and the second polariser (analyser) after the sample. Images were taken whereby maximum polarisation was achieved by adjustment of the polarising filters, and again orthogonal to this maximum polarisation. The two captured images were merged using the MAX function in ImageJ software (freely available from https://imagej.nih.gov/) which enables a complete visualisation of the collagen fibres present [18]. The total area covered by collagen was first calculated in number of pixels using ImageJ. This was achieved excluding the dark background of the images and with colour thresholding, selecting consistent hue ranges to quantify the areas of collagen that polarised red, orange, yellow or green within each pixel. The recorded data was input into the following equations:

$$\% \text{ Red Fibres} = (\text{Red Pixels})/(\text{Total Collagen Pixels} \times 0.01)$$

$$\% \text{ Orange Fibres} = (\text{Orange Pixels})/(\text{Total Collagen Pixels} \times 0.01)$$

$$\% \text{ Yelow Fibres} = (\text{Yellow Pixels})/(\text{Total Collagen Pixels} \times 0.01)$$

$$\% \text{ Green Fibres} = (\text{Green Pixels})/(\text{Total Collagen Pixels} \times 0.01)$$

The isotropy of the polarised collagen fibres was evaluated using the OrientationJ plugin on ImageJ. This method can characterize the orientation and isotropy properties of a region of interest (ROI) in an image, based on the evaluation of the gradient structure tensor and in particular analyse collagen orientation (direction) and the degree to which a dominant direction exists in a tissue sample [19]. The coherency indicates if the local image features are oriented or not: C is 1 when the local structure has one dominant orientation and C is 0 if the image is essentially isotropic in the local neighbourhood.

*Quantitative analysis of blood vessels*

Immunohistochemical staining was performed with CD31 (ab28364, Abcam) (1:200). Slides were incubated for 1 hour at 37 °C. A mouse and rabbit specific HRP/DAB kit (ABC) detection IHC kit (ab64264, Abcam) was used. Sections were counterstained with haematoxylin, dehydrated through a series of graded alcohols, cleared in xylene and coverslipped using DPX. Slides were imaged using an Olympus SlideScanner VS120 with an UPLSAPO 20× objective lens. Analysis of neo-vascularisation: Number per area (Na) and radial diffusion distance (Rd) were all calculated using an unbiased counting frame [15, 16]. This counting frame consists of red forbidden lines and green acceptance lines. Any blood vessel that is cut by a forbidden line is not counted. Blood vessels that appear outside the counting frame or are cut by the acceptance lines without also cutting the forbidden line are counted. The application of this counting formula generates an unbiased estimate of the number of blood vessels per unit area. Na = cN/(cPts x Area of Grid) taking cN as the cumulative no.of blood vessels counted and cPts as the cumulative no. of points. The length density (Lv) of a blood vessel is the length of blood vessels present per unit volume and is calculated by multiplying the numerical density by 2. From length density the radial diffusion distance (Rd) can be calculated by the following formulas: Lv=Na x 2, Rd=$1/\sqrt{(\pi(lv))}$

## Equivalents

The foregoing description details presently preferred embodiments of the present invention. Numerous modifications and variations in practice thereof are expected to occur to those skilled in the art upon consideration of these descriptions. The invention is defined by the claims appended hereto.

## References

1. Lotti, F., et al., Invasive Intraneural Interfaces: Foreign Body Reaction Issues. Frontiers in neuroscience, 2017. 11: p. 497-497.
2. Kozai, T.D.Y., et al., Brain tissue responses to neural implants impact signal sensitivity and intervention strategies. ACS chemical neuroscience, 2014. 6(1): p. 48-67.
3. Cetin, N., et al., Foreign Body Reaction to Dialysis Chatheter and Peritoneal Fluid Eosinophilia in a Child on Continuous Ambulatory Peritoneal Dialysis. Iran J Kidney Dis, 2017. 11(4): p. 319-321.
4. Liu, X., et al., Comparison of the postoperative incidence rate of capsular contracture among different breast implants: a cumulative meta-analysis. PloS one, 2015. 10(2): p. e0116071.
5. Tarakji, K.G., et al., Cardiac implantable electronic device infection in patients at risk. Arrhythmia & electrophysiology review, 2016. 5(1): p. 65.
6. Sharkawy, A.A., et al., Engineering the tissue which encapsulates subcutaneous implants. I. Diffusion properties. Journal of Biomedical Materials Research Part A, 1997. 37(3): p. 401-412.
7. Novak, M.T., F. Yuan, and W.M. Reichert, Modeling the relative impact of capsular tissue effects on implanted glucose sensor time lag and signal attenuation. Analytical and bioanalytical chemistry, 2010. 398(4): p. 1695-1705.
8. Thome-Duret, V., et al., Modification of the sensitivity of glucose sensor implanted into subcutaneous tissue. Diabetes & Metabolism, 1996. 22(3): p. 174-178.
9. Fritschy, W.M., et al., The capsular overgrowth on microencapsulated pancreatic islet graft in streptozotocin and autoimmune diabetic rats. Transplant international, 1994. 7(4): p. 264-271.
10. Soon-Shiong, P., et al. An immunologic basis for the fibrotic reaction to implanted microcapsules. in Transplantation proceedings. 1991.
11. Sharkawy, A.A., et al., Engineering the tissue which encapsulates subcutaneous implants. III. Effective tissue response times. Journal of Biomedical Materials Research Part A, 1998. 40(4): p. 598-605.
12. Sharkawy, A.A., et al., Engineering the tissue which encapsulates subcutaneous implants. II. Plasma-tissue exchange properties. Journal of Biomedical Materials Research Part A, 1998. 40(4): p. 586-597.
13. Whyte, W., et al., Sustained release of targeted cardiac therapy with a replenishable implanted epicardial reservoir. Nature Biomedical Engineering, 2018.
14. Howard, C. and M. Reed, Unbiased Stereology, 2nd Edn. Liverpool. 2010, QTP Publications.
15. Jensen, E. and H. Gundersen, The stereological estimation of moments of particle volume. Journal of applied probability, 1985. 22(1): p. 82-98.
16. Dockery, P. and J. Fraher, The quantification of vascular beds: a stereological approach. Experimental and molecular pathology, 2007. 82(2): p. 110-120.
17. Monaghan, M.G., et al., Exogenous miR-29B Delivery Through a Hyaluronan-Based Injectable System Yields Functional Maintenance of the Infarcted Myocardium. Tissue Eng Part A, 2018. 24(1-2): p. 57-67.

18. Coleman, R., Picrosirius red staining revisited. Acta Histochem, 2011. 113(3): p. 231-3.

19. Rezakhaniha, R., et al., Experimental investigation of collagen waviness and orientation in the arterial adventitia using confocal laser scanning microscopy. Biomechanics and Modeling in Mechanobiology, 2012. 11(3): p. 461-473.

20. Roche, E.T., et al., Soft robotic sleeve supports heart function. Science translational medicine, 2017. 9(373): p. eaaf3925.

21. Horvath, M.A., et al., An Intracardiac Soft Robotic Device for Augmentation of Blood Ejection from the Failing Right Ventricle. Annals of Biomedical Engineering, 2017. 45(9): p. 2222-2233.

## Claims

1. An implantable medical device having a soft tissue interfacing surface comprising at least one actuatable capsule having a soft tissue interfacing deflectable membrane configured for cyclical deflection upon actuation of the capsule, in which the at least one actuatable capsule is a soft polymer capsule configured for pneumatic, hydraulic, electrical, or ultrasound actuation to cause cyclical deflection of the soft-tissue interfacing membrane to modulate the biomechanics of the soft tissue interface during use by altering one or more of displacement, force, strain, fluid flow and cellular activity in peri-implant tissue at the soft tissue interface so as to reduce or inhibit fibrotic encapsulation of the implantable medical device during use, in which the soft-tissue interfacing deflectable membrane is porous or configured to become porous upon deflection.

2. An implantable medical device according to Claim 1, in which the at least one actuatable capsule comprises:

   an actuation chamber containing a first fluid;
   a therapeutic chamber containing a second fluid;
   a deflectable membrane separating the actuation chamber and therapeutic chamber; and
   an actuation conduit in fluidic communication with the actuation chamber for fluidic actuation of the actuation chamber,
   wherein the therapeutic chamber comprises the soft tissue interfacing deflectable membrane

3. An implantable medical device according to Claim 1 or 2 configured for actuation of the actuation chamber at a frequency of 0.1 to 10 Hz.

4. An implantable medical device according to Claim 2 configured for actuation of the actuation chamber at an actuation pressure of 3,347 to 137,895 N/m2 (0.5 to 20.0 PSI).

5. An implantable medical device according to Claim 2 configured for actuation of the actuation chamber at an actuation pressure of 3,347 to 137,895 N/m2 (0.5 to 20.0 PSI).

6. An implantable medical device according to any preceding Claim, in which the soft tissue interfacing surface comprises a plurality of actuatable capsules.

7. An implantable medical device according to any preceding Claim, in which the at least one actuatable capsule is a millisized capsule.

8. An implantable medical device according to any preceding Claim, having a primary function that is independent of the cyclical deflection of the soft tissue interfacing deflectable membrane.

9. An implantable medical device according to any preceding Claim, selected from a breast implant, an indwelling catheter, a pacemaker, a biosensor, a cell or drug delivery device, and a neural probe.

10. An implantable medical device according to any preceding Claim, in which the soft tissue interfacing surface of the medical device is provided by a cover or sheath configured for retro-fitting to the medical device and comprising the at least one actuatable capsule, and in which the soft tissue interfacing deflectable membrane of the or each actuatable capsule is substantially flush with an external surface of the cover or sheath.

11. An implantable medical device according to any preceding Claim, in which the or each capsule is dome shaped having a flat base provided by the soft tissue interfacing deflectable membrane.

**12.** An implantable medical device according to Claim 2, in which the or each capsule comprises a therapeutic conduit in fluidic communication with the therapeutic chamber for delivery of an active agent.

**13.** An implantable medical device according to Claim 2, including a pump operatively connected to the actuation conduit.

**14.** An implantable medical device according to Claim 13, in which the or each capsule comprises a second actuation conduit in fluidic communication with the actuation chamber, wherein fluid is pumped by the pump into the actuation chamber through the actuation conduit and returned to the pump through the second conduit, in which the second conduit optionally comprises a fluidic resistor configured to resist fluid flow and an expandable chamber distal of the fluidic resistor.

**15.** An implantable medical device according to Claim 1, including:

an actuator for causing cyclical deflection of the soft tissue interfacing deflectable membrane; and
a controller to modify output parameters of the actuator.

**Patentansprüche**

**1.** Implantierbare medizinische Vorrichtung mit einer Weichgewebe berührenden Oberfläche, umfassend mindestens eine betätigbare Kapsel mit einer Weichgewebe berührenden auslenkbaren Membran, die zur zyklischen Auslenkung bei Betätigung der Kapsel konfiguriert ist, wobei die mindestens eine betätigbare Kapsel eine Weichpolymerkapsel ist, die zur pneumatischen, hydraulischen, elektrischen oder Ultraschallbetätigung konfiguriert ist, um eine zyklische Auslenkung der Weichgewebe berührenden Membran zu bewirken, um die Biomechanik der Weichgewebeberührungsfläche während des Einsatzes durch Ändern eines oder mehrerer von Verschiebung, Kraft, Dehnung, Fluidfluss und Zellaktivität in Peri-Implantatgewebe an der Weichgewebeberührungsfläche zu modulieren, um eine Kapselfibrose um die implantierbare medizinische Vorrichtung während des Einsatzes zu verringern oder zu verhindern, wobei die Weichgewebe berührende auslenkbare Membran porös oder dazu konfiguriert ist, bei Auslenkung porös zu werden.

**2.** Implantierbare medizinische Vorrichtung nach Anspruch 1, wobei die mindestens eine betätigbare Kapsel Folgendes umfasst:

eine Betätigungskammer, die ein erstes Fluid enthält;
eine therapeutische Kammer, die ein zweites Fluid enthält;
eine auslenkbare Membran, die die Betätigungskammer und die therapeutische Kammer trennt; und
eine Betätigungsleitung in fluidischer Verbindung mit der Betätigungskammer zur fluidischen Betätigung der Betätigungskammer,
wobei die therapeutische Kammer die Weichgewebe berührende auslenkbare Membran umfasst.

**3.** Implantierbare medizinische Vorrichtung nach Anspruch 1 oder 2, die zur Betätigung der Betätigungskammer mit einer Frequenz von 0,1 bis 10 Hz konfiguriert ist.

**4.** Implantierbare medizinische Vorrichtung nach Anspruch 2, die zur Betätigung der Betätigungskammer bei einem Betätigungsdruck von 3.347 bis 137.895 N/m2 (0,5 bis 20,0 psi) konfiguriert ist.

**5.** Implantierbare medizinische Vorrichtung nach Anspruch 2, die zur Betätigung der Betätigungskammer bei einem Betätigungsdruck von 3.347 bis 137.895 N/m2 (0,5 bis 20,0 psi) konfiguriert ist.

**6.** Implantierbare medizinische Vorrichtung nach einem der vorangehenden Ansprüche, wobei die Weichgewebe berührende Oberfläche eine Vielzahl von betätigbaren Kapseln umfasst.

**7.** Implantierbare medizinische Vorrichtung nach einem der vorangehenden Ansprüche, wobei die mindestens eine Kapsel in Milligröße ist.

**8.** Implantierbare medizinische Vorrichtung nach einem der vorangehenden Ansprüche mit einer Hauptfunktion, die von der zyklischen Auslenkung der Weichgewebe berührenden auslenkbaren Membran unabhängig ist.

**9.** Implantierbare medizinische Vorrichtung nach einem der vorangehenden Ansprüche, ausgewählt aus einem Brustimplantat, einem Verweilkatheter, einem Schrittmacher, einem Biosensor, einer Zellen- oder Blutzuführvorrichtung und einer Neuralsonde.

**10.** Implantierbare medizinische Vorrichtung nach einem der vorangehenden Ansprüche, wobei die Weichgewebe berührende Oberfläche der medizinischen Vorrichtung von einer Abdeckung oder Hülle bereitgestellt ist, die zum nachträglichen Anbringen an der medizinischen Vorrichtung konfiguriert ist und die mindestens eine betätigbare Kapsel umfasst und wobei die Weichgewebe berührende auslenkbare Membran der oder jeder betätigbaren Kapsel mit einer äußeren Oberfläche der Abdeckung oder Hülle im Wesentlichen bündig ist.

**11.** Implantierbare medizinische Vorrichtung nach einem der vorangehenden Ansprüche, wobei die oder jede Kapsel kuppelförmig mit einem von der Weichgewebe berührenden auslenkbaren Membran bereitgestellten flachen Boden ist.

**12.** Implantierbare medizinische Vorrichtung nach Anspruch 2, wobei die oder jede Kapsel eine therapeutische Leitung in fluidischer Verbindung mit der therapeutischen Kammer zum Zuführen eines Wirkstoffs umfasst.

**13.** Implantierbare medizinische Vorrichtung nach Anspruch 2, umfassend eine Pumpe in Wirkverbindung mit der Betätigungsleitung.

**14.** Implantierbare medizinische Vorrichtung nach Anspruch 13, wobei die oder jede Kapsel eine zweite Betätigungsleitung in fluidischer Verbindung mit der Betätigungskammer umfasst, wobei Fluid von der Pumpe durch die Betätigungsleitung hindurch in die Betätigungskammer gepumpt wird und durch die zweite Leitung hindurch zu der Pumpe zurückgeführt wird, wobei die zweite Leitung optional einen Fluidwiderstand, der dazu konfiguriert ist, Fluidfluss zu widerstehen, und eine ausdehnbare Kammer distal des fluidischen Widerstands umfasst.

**15.** Implantierbare medizinische Vorrichtung nach Anspruch 1, umfassend:

einen Aktor zum Bewirken zyklischer Auslenkung der Weichgewebe berührenden auslenkbaren Membran; und eine Steuerung zum Modifizieren von Ausgangsparametern des Aktors.

**Revendications**

**1.** Dispositif médical implantable ayant une surface d'interface avec un tissu mou comprenant au moins une capsule actionnable ayant une membrane capable de déflexion d'interface avec un tissu mou configurée pour une déflexion cyclique lors de l'actionnement de la capsule, dans lequel l'au moins une capsule actionnable est une capsule de polymère souple configurée pour un actionnement pneumatique, hydraulique, électrique, ou par ultrasons pour provoquer une déflexion cyclique de la membrane d'interface avec un tissu mou afin de moduler la biomécanique de l'interface avec un tissu mou pendant l'utilisation en modifiant un ou plusieurs éléments parmi le déplacement, la force, la contrainte, l'écoulement de fluide et l'activité cellulaire dans un tissu péri-implantaire au niveau de l'interface avec un tissu mou de manière à réduire ou inhiber l'encapsulation fibreuse du dispositif médical implantable pendant l'utilisation, dans lequel la membrane capable de déflexion d'interface avec un tissu mou est poreuse ou configurée pour devenir poreuse lors de la déflexion.

**2.** Dispositif médical implantable selon la revendication 1, dans lequel l'au moins une capsule actionnable comprend :

une chambre d'actionnement contenant un premier fluide ;
une chambre thérapeutique contenant un deuxième fluide ;
une membrane capable de déflexion séparant la chambre d'actionnement et la chambre thérapeutique ; et
un conduit d'actionnement en communication fluidique avec la chambre d'actionnement pour un actionnement fluidique de la chambre d'actionnement,
dans lequel la chambre thérapeutique comprend la membrane capable de déflexion d'interface avec un tissu mou.

**3.** Dispositif médical implantable selon la revendication 1 ou 2 configuré pour un actionnement de la chambre d'actionnement à une fréquence de 0,1 à 10 Hz.

4. Dispositif médical implantable selon la revendication 2 configuré pour un actionnement de la chambre d'actionnement à une pression d'actionnement de 3 347 à 137 895 N/m2 (0,5 à 20,0 psi).

5. Dispositif médical implantable selon la revendication 2 configuré pour un actionnement de la chambre d'actionnement à une pression d'actionnement de 3 347 à 137 895 N/m2 (0,5 à 20,0 psi).

6. Dispositif médical implantable selon l'une quelconque des revendications précédentes, dans lequel la surface d'interface avec un tissu mou comprend une pluralité de capsules actionnables.

7. Dispositif médical implantable selon l'une quelconque des revendications précédentes, dans lequel l'au moins une capsule actionnable est une capsule millimétrique.

8. Dispositif médical implantable selon l'une quelconque des revendications précédentes, ayant une fonction primaire qui est indépendante de la déflexion cyclique de la membrane capable de déflexion d'interface avec un tissu mou.

9. Dispositif médical implantable selon l'une quelconque des revendications précédentes, choisi parmi un implant mammaire, un cathéter à demeure, un stimulateur cardiaque, un biocapteur, un dispositif de délivrance de cellules ou de médicaments, et une sonde neuronale.

10. Dispositif médical implantable selon l'une quelconque des revendications précédentes, dans lequel la surface d'interface avec un tissu mou du dispositif médical est fournie par une couverture ou une gaine configurée pour être ajustée de manière rétroactive sur le dispositif médical et comprenant l'au moins une capsule actionnable, et dans lequel la membrane capable de déflexion d'interface avec un tissu mou de la ou de chaque capsule actionnable est sensiblement au même niveau qu'une surface externe de la couverture ou de la gaine.

11. Dispositif médical implantable selon l'une quelconque des revendications précédentes, dans lequel la ou chaque capsule est en forme de dôme ayant une base plate fournie par la membrane capable de déflexion d'interface avec un tissu mou.

12. Dispositif médical implantable selon la revendication 2, dans lequel la ou chaque capsule comprend un conduit thérapeutique en communication fluidique avec la chambre thérapeutique pour la délivrance d'un agent actif.

13. Dispositif médical implantable selon la revendication 2, incluant une pompe connectée de manière fonctionnelle au conduit d'actionnement.

14. Dispositif médical implantable selon la revendication 13, dans lequel la ou chaque capsule comprend un deuxième conduit d'actionnement en communication fluidique avec la chambre d'actionnement, dans lequel le fluide est pompé par la pompe jusque dans la chambre d'actionnement à travers le conduit d'actionnement et renvoyé à la pompe à travers le deuxième conduit, dans lequel le deuxième conduit comprend facultativement une résistance fluidique configurée pour résister à l'écoulement de fluide et une chambre capable de déploiement distale par rapport à la résistance fluidique.

15. Dispositif médical implantable selon la revendication 1, incluant :

un actionneur destiné à provoquer une déflexion cyclique de la membrane capable de déflexion d'interface avec un tissu mou ; et
un dispositif de commande pour modifier des paramètres de sortie de l'actionneur.

FIGURE 1A

FIGURE 1B

OPTION B • **POROUS** • For Therapy Delivery

**① OUTER BALLOON** 12 mil TPU

**② INNER BALLOON** 3mil TPU

**③ MEMBRANE** 3 mil TPU

**THERAPY DELIVERY CATHETER**

**ACTUATION CATHETER**

**④ CATHETER**

EXPLODED VIEW

**Pressure = atm**

Therapy Delivery Catheter · Actuation Reservoir · Middle Membrane · Outer Membrane

Therapy Reservoir

Actuation Catheter · Membrane · **HOST TISSUE**

Suture

**flat membrane**

**Pressure > atm** Actuation

FLUID

**convex membrane**

**Pressure = atm** Therapy delivery

THERAPY

**flat membrane**

# FIGURE 1C

**FIGURE 2A**

**FIGURE 2B**

Mises Stress (MPa)

**FIGURE 2C**

Max In-Plane Strain

**FIGURE 2D**

## FIGURE 2E

## FIGURE 2F

**FIGURE 2G**

**FIGURE 2H**

**1** MAKE 1 CM INCISION AT BASE OF NECK BETWEEN SHOULDER BLADES

**2** MEASURE 7 CM FROM INCISION ALONG BACK OF ANIMAL. 1 CM EITHERSIDE OF THE SPINE

**3** MEASURE 9CM FROM INCISION ALONG BACK OF ANIMAL. 1 CM EITHER SIDE OF THE SPINE

**4** MAKE THE PLACEMENT INCISION ON THE RIGHTSIDE OF THE ANIMAL TO CREATE THE SUBCUTANEOUS POCKET FOR DEVICE 1

**5** MAKE A BLUNT DISSECTION

**6** REPEAT STEPS 4 & 5 ON THE LEFT SIDE

**7** PLACE SUBCUTANEOUS PORT IN POSITION AT BASE OF NECK AND FEED THE DEVICES THROUGH THE SUBCUTANEOUS TUNNELS

**8** PLACE A SUTURE THROUGH EACH SIDE OF THE SUBCUTANEOUS PORT AND UNDERLYING MUSCLE TO SECURE

**9** PLACE EXPANDER IN THE RIGHT INCISION TO MAKE THE DEVICE RESERVOIR CLEARLY VISIBLE. BRING A SUTURE THROUGH THE SUTURE HOLE AND UNDERLYING MUSCLE TO SECURE IN PLACE

**10** REPEAT STEP 9 ON THE LEFT

**11** CLOSE THE PLACEMENT INCISION WITH INTERRUPTED BRAIDED SUTURE EACH SIDE OF THE PORT

**12** CLOSE THE SUBCUTANEOUS PORT INCISIONS WTH BRAIDED SUTURES

**FIGURE 3A**

| Experimental group | Experimental timeline |
|---|---|
| Non-porous (- actuation) n=6 | |
| Non-porous (+ actuation) n=6 | Actuation – regime 1 or 2 |
| Porous (- actuation) n=3 | |
| Porous (+ actuation) n=3 | Actuation – regime 2 |

D-1  D0  D3  D8  D14

Implant    IVIS    IVIS    IVIS

D15

Actuation:

D0-D14: 1 Hz for 5 mins every 12H

Regime 1: 1psi input pressure

Regime 2: 2 psi input pressure

Adrenaline
delivery

**Post-sacrifice**

microCT

Histology

Immunohistochemistry

## FIGURE 3B

Blue = Device Capsule    Purple = Fibrotic

FIGURE 4A

FIGURE 4B

**FIGURE 4C**

**FIGURE 5A**

**FIGURE 5B**

**FIGURE 5C**

**FIGURE 5D**

**FIGURE 5E**

**FIGURE 5F**

**FIGURE 5G**

**FIGURE 5H**

**FIGURE 5I**

**FIGURE 5J**

**FIGURE 6**

| Experimental group | Experimental timeline | | Actuation: |
|---|---|---|---|
| Porous (- actuation) n=3 | Control | | D0-D14: 2 psi input pressure at 1 Hz for 5 mins every 12H |
| Porous (+ actuation) n=3 | Actuation | | |

D-1 Implant · D0 · D8 IVIS · D14 IVIS · D15 Adrenaline delivery

## FIGURE 7A

0 min    30 min    60 min

Ctrl   Act

X10⁹
Radiant Efficiency

## FIGURE 7B

□ Control
□ Actuated

AUC (a.u.)

Day 8    Day 14

## FIGURE 7C

**FIGURE 7D**

**FIGURE 7E**

FIGURE 7F

FIGURE 8A

Electropneumatics and control

## FIGURE 8B

## FIGURE 8C

**FIGURE 9A**

**FIGURE 9B**

$$U = \sum_{i=1}^{N} \frac{2\mu_i}{\alpha_i^2} \left( \bar{\lambda}_1^{\alpha_i} + \bar{\lambda}_2^{\alpha_i} + \bar{\lambda}_3^{\alpha_i} - 3 \right) + \sum_{i=1}^{N} \frac{1}{D_i} \left( J^{el} - 1 \right)^{2i},$$

**FIGURE 9C**

**FIGURE 9D**

**FIGURE 9E**

S, Mises
SNEG, (fraction = -1.0)
(Avg: 75%)

1.800
1.650
1.500
1.350
1.200
1.050
0.900
0.750
0.600
0.450
0.300
0.150
0.000

Y
X
Z

Non-porous membrane

S, Mises
SNEG, (fraction = -1.0)
(Avg: 75%)

2.086
1.800
1.650
1.500
1.350
1.200
1.050
0.900
0.750
0.600
0.450
0.300
0.150
0.000

Y
X
Z

Porous membrane

**FIGURE 9F**

LE, Max. In-Plane Principal
SNEG, (fraction = -1.0)
(Avg: 75%)

Non-porous membrane

LE, Max. In-Plane Principal
SNEG, (fraction = -1.0)
(Avg: 75%)

Porous membrane

**FIGURE 9G**

# BALLOON THERMOFORMING

## Equipment

THERMOFORMER

POSITIVE MOULD

## Process

(1) place TPU sheet

(2) Secure in place

(3) bring TPU up

(4) turn on Heat

(5) heat the TPU

(6) bring heated TPU over mould

(7) Turn on Vacuum

Take mould out of thermoformer

## FIGURE 10A

# HEAT SEALING

## Equipment

HEAT PRESS

NEGATIVE MOULD

## Process

(1) Cut both 12 mil and 3 mil positive molds

(2) place 12 mil outer balloon in negative mould first

(3) place 3 mil inner balloon in negative mould

(4) place sheet of teflon on top

(5) bring down heat arm of heat press

(6) heat for 13 seconds at 330 F/160 C

(7) Remove Heat and Teflon

(8) Completed Device

**FIGURE 10B**

FIGURE 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014236210 A
- US 4718893 A

- US 5707361 A

**Non-patent literature cited in the description**

- **LOTTI, F. et al.** Invasive Intraneural Interfaces: Foreign Body Reaction Issues. *Frontiers in neuroscience*, 2017, vol. 11, 497-497
- **KOZAI, T.D.Y. et al.** Brain tissue responses to neural implants impact signal sensitivity and intervention strategies. *ACS chemical neuroscience*, 2014, vol. 6 (1), 48-67
- **CETIN, N. et al.** Foreign Body Reaction to Dialysis Chatheter and Peritoneal Fluid Eosinophilia in a Child on Continuous Ambulatory Peritoneal Dialysis. *Iran J Kidney Dis*, 2017, vol. 11 (4), 319-321
- **LIU, X. et al.** Comparison of the postoperative incidence rate of capsular contracture among different breast implants: a cumulative meta-analysis. *PloS one*, 2015, vol. 10 (2), e0116071
- **TARAKJI, K.G. et al.** Cardiac implantable electronic device infection in patients at risk. *Arrhythmia & electrophysiology review*, 2016, vol. 5 (1), 65
- **SHARKAWY, A.A. et al.** Engineering the tissue which encapsulates subcutaneous implants. I. Diffusion properties. *Journal of Biomedical Materials Research Part A*, 1997, vol. 37 (3), 401-412
- **NOVAK, M.T.** ; **F. YUAN** ; **W.M. REICHERT**. Modeling the relative impact of capsular tissue effects on implanted glucose sensor time lag and signal attenuation. *Analytical and bioanalytical chemistry*, 2010, vol. 398 (4), 1695-1705
- **THOME-DURET, V. et al.** Modification of the sensitivity of glucose sensor implanted into subcutaneous tissue. *Diabetes & Metabolism*, 1996, vol. 22 (3), 174-178
- **FRITSCHY, W.M. et al.** The capsular overgrowth on microencapsulated pancreatic islet graft in streptozotocin and autoimmune diabetic rats. *Transplant international*, 1994, vol. 7 (4), 264-271
- **SOON-SHIONG, P. et al.** An immunologic basis for the fibrotic reaction to implanted microcapsules. *Transplantation proceedings*, 1991

- **SHARKAWY, A.A. et al.** Engineering the tissue which encapsulates subcutaneous implants. III. Effective tissue response times. *Journal of Biomedical Materials Research Part A*, 1998, vol. 40 (4), 598-605
- **SHARKAWY, A.A. et al.** Engineering the tissue which encapsulates subcutaneous implants. II. Plasma-tissue exchange properties. *Journal of Biomedical Materials Research Part A*, 1998, vol. 40 (4), 586-597
- **WHYTE, W. et al.** Sustained release of targeted cardiac therapy with a replenishable implanted epicardial reservoir. *Nature Biomedical Engineering*, 2018
- **HOWARD, C.** ; **M. REED**. Unbiased Stereology. QTP Publications, 2010
- **JENSEN, E.** ; **H. GUNDERSEN**. The stereological estimation of moments of particle volume. *Journal of applied probability*, 1985, vol. 22 (1), 82-98
- **DOCKERY, P.** ; **J. FRAHER**. The quantification of vascular beds: a stereological approach. *Experimental and molecular pathology*, 2007, vol. 82 (2), 110-120
- **MONAGHAN, M.G. et al.** Exogenous miR-29B Delivery Through a Hyaluronan-Based Injectable System Yields Functional Maintenance of the Infarcted Myocardium. *Tissue Eng Part A*, 2018, vol. 24 (1-2), 57-67
- **COLEMAN, R.** Picrosirius red staining revisited. *Acta Histochem*, 2011, vol. 113 (3), 231-3
- **REZAKHANIHA, R. et al.** Experimental investigation of collagen waviness and orientation in the arterial adventitia using confocal laser scanning microscopy. *Biomechanics and Modeling in Mechanobiology*, 2012, vol. 11 (3), 461-473
- **ROCHE, E.T. et al.** Soft robotic sleeve supports heart function. *Science translational medicine*, 2017, vol. 9 (373), 3925
- **HORVATH, M.A. et al.** An Intracardiac Soft Robotic Device for Augmentation of Blood Ejection from the Failing Right Ventricle. *Annals of Biomedical Engineering*, 2017, vol. 45 (9), 2222-2233